# EUROPEAN PATENT APPLICATION

(11) **EP 4 154 870 A1**
(43) Date of publication of application: **29.03.2023**
(21) Application number: 21809116.3
(22) Date of filing: 24.05.2021
(51) Int. Cl.: A61K 9/08, A61K 47/26, A61K 47/18, A61K 47/12, A61K 47/22, A61K 47/20, A61K 47/64, A61K 38/00, A61K 38/17, A61K 38/26

(54) **LIQUID PREPARATION OF LONG-ACTING CONJUGATE OF GLUCAGON/GLP-1/GIP TRIGONAL AGONIST**

(30) Priority: 22.05.2020 KR 20200061875
(71) Applicant: Hanmi Pharm. Co., Ltd., Hwaseong-si, Gyeonggi-do 18536 (KR)
(72) Inventor: LIM, Hyung Kyu, Hwaseong-si, Gyeonggi-do 18469 (KR); KIM, Sang Yun, Hwaseong-si, Gyeonggi-do 18469 (KR); BAE, Sung Min, Hwaseong-si, Gyeonggi-do 18469 (KR)
(74) Representative: Mewburn Ellis LLP
(86) International application number: PCT/KR2021/006462
(87) International publication number: WO 2021/235916

(57) **Abstract**

The present invention relates to a liquid formulation of a long-acting conjugate of glucagon, GLP-1, and GIP trigonal agonist, and a method for preparing the same.

## Description

### [Technical Field]

The present invention relates to a liquid formulation of a long-acting conjugate of glucagon/GLP-1/GIP trigonal agonist, and a method of preparing the liquid formulation.

### [Background Art]

Obesity and diabetes including type 2 diabetes are representative metabolic diseases that occur in modern society. These diseases are regarded as health-threatening factors in the world, and the accompanying economic costs due to the incidence of these diseases are rapidly increasing at present.

Glucagon-like peptide-1 (GLP-1) and glucose-dependent insulinotropic polypeptide (GIP) are representative gastrointestinal hormones and neuronal hormones and are materials involved in the control of blood glucose levels according to food intake. Glucagon is a peptide hormone secreted by the pancreas and is involved in controlling the blood glucose levels along with the two materials described above.

GLP-1 is a hormone secreted by the small intestine stimulated by food intake. GLP-1 promotes insulin secretion in the pancreas in a blood glucose-dependent manner and inhibits the secretion of glucagon, thus helping the action of lowering blood glucose levels. Additionally, GLP-1 has the roles of slowing digestive action in the gastrointestinal tract by acting as a satiety factor, and reducing the amount of food intake by delaying the time for emptying digested food in the gastrointestinal tract. Furthermore, the administration of GLP-1 to rats was reported to have effects of inhibiting food intake and reducing body weight, and these effects were confirmed to occur equally both in normal and obese states, thus showing the potential of GLP-1 as an agent for treating obesity.

GIP, one of the gastrointestinal hormones secreted by the stimulation of food intake, as is the case of GLP-1, is a hormone consisting of 42 amino acids secreted by intestinal K-cells. GIP was reported to perform the functions of promoting the secretion of insulin in the pancreas in a blood glucose-dependent manner and helping to lower the blood glucose levels, thereby exhibiting effects of increasing the activation of GLP-1, anti-inflammation, *etc.*

Glucagon is produced in the pancreas when the blood glucose levels fall due to reasons such as medications, diseases, deficiency in hormones or enzymes, *etc.* Glucagon sends a signal for glycogen breakdown in the liver to induce the release of glucose and increases blood glucose levels to a normal level. In addition to the effect of increasing the blood glucose levels, glucagon suppresses appetite in animals and humans and activates hormone-sensitive lipase of adipocytes to promote lipolysis and energy expenditure, thereby showing an anti-obesity effect.

As such, active studies are being conducted to develop GLP-1 as a therapeutic agent for treating diabetes and obesity, based on the effects of GLP-1 in controlling blood glucose levels and reducing body weight. Currently, exendin-4, prepared from lizard venom and having an amino acid homology of about 50% with GLP-1, is under development as a therapeutic agent for treating the same kinds of diseases. However, therapeutic agents containing GLP-1 and exendin-4 were reported to show side-effects such as vomiting and nausea (Syed Y. Y., Drugs, 2015 July; 75(10):1141-52).

Accordingly, there is currently a novel material under development which can highly activate GLP-1, GIP, and glucagon receptors and which has effects of controlling blood glucose levels and reducing body weight without causing any side-effects such as vomiting and nausea. Additionally, there is currently a novel material under development which has various active ratios to GLP-1, GIP, and glucagon receptors. For example, a material which has an effect of reducing body weight but has a significantly higher effect of controlling blood glucose levels due to high GLP-1 and GIP activities but with relatively low glucagon activity for a hypoglycemic effect; or a material which has high activities for all of GLP-1, GIP, and glucagon, thus having a significantly high effect of reducing body weight.

### [Disclosure]

### [Technical Problem]

There is a need for the development of a stable liquid formulation capable of storing the long-acting conjugate of a peptide having activities for all of the developed glucagon receptor, GLP-1 receptor, and GIP receptor for a long time without concerning virus contamination.

### [Technical Solution]

It is one object of the present invention to provide a liquid formulation of a long-acting conjugate of glucagon, GLP-1, and GIP trigonal agonist.

It is another object of the present invention to provide a method for preparing the liquid formulation.

### [Advantageous Effects]

The liquid formulation according to the present invention has the economic advantage by providing storage stability to the conjugate of the present invention having a large molecular weight in a simple formulation.

### [Brief Description of Drawings]

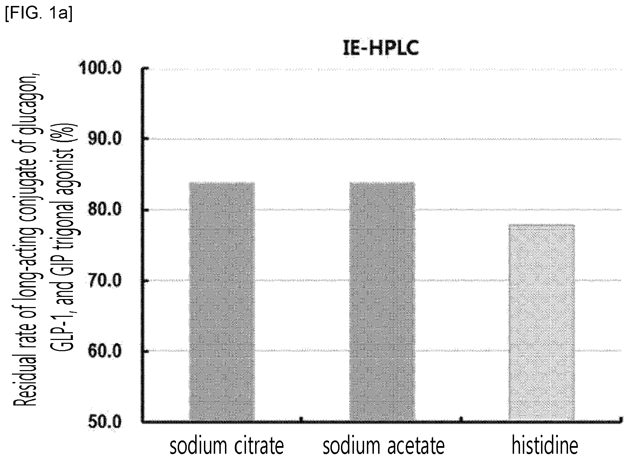

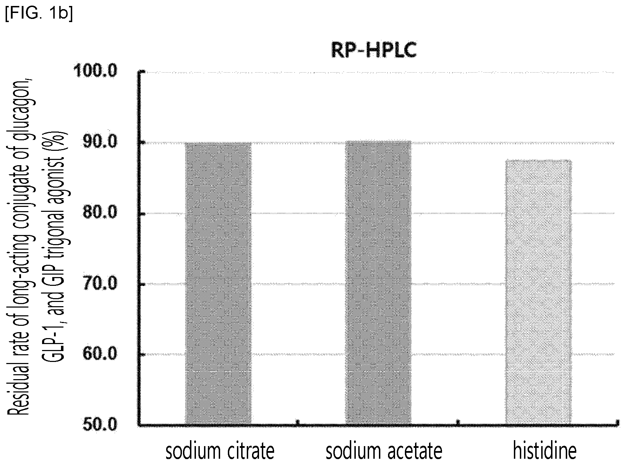

FIGS. 1A and 1B are the result of confirming the stability of the long-acting conjugate of glucagon, GLP-1, and GIP trigonal agonist according to the types of buffering agents based on the liquid formulation of Example 3 (sodium citrate, pH 5.5, mannitol, polysorbate 20, methionine). Specifically, the each of the compositions shown in Table 9 were used as the liquid formulation of a long-acting conjugate of glucagon, GLP-1, and GIP trigonal agonist, and the stability results were shown after storage at 25°C for 6 weeks.

### [Detailed Description of the Invention]

One aspect for implementing the present invention provides a liquid formulation of a long-acting conjugate of glucagon, GLP-1 (Glucagon-like peptide-1), and GIP (Glucose-dependent insulinotropic polypeptide) trigonal agonist. The long-acting conjugate refers to a substance in which a peptide having activities for a glucagon receptor, a GLP-1 receptor, and a GIP receptor is covalently bonded to an immunoglobulin Fc fragment by a linker.

In one embodiment, the present invention relates to a liquid formulation of a long-acting conjugate of a peptide having activities for a glucagon receptor, a GLP-1 receptor, and a GIP receptor, wherein the liquid formulation includes: the long-acting conjugate of a peptide having activities for a glucagon receptor, a GLP-1 receptor, and a GIP receptor in a pharmacologically effective amount; and i) a buffering agent, and ii) an albumin-free stabilizer including sugar alcohol, saccharide, or a combination thereof. The long-acting conjugate may refer to a substance in which a peptide having activities for a glucagon receptor, a GLP-1 receptor, and a GIP receptor is covalently bonded to an immunoglobulin Fc fragment by a linker.

In one embodiment, the liquid formulation is a liquid formulation including the long-acting conjugate of Chemical Formula 1 below; a buffering agent; and sugar alcohol, saccharide, or a combination thereof:

[Chemical Formula 1] Q - Lₐ - Z

In Chemical Formula 1 above,
Q is a peptide of General Formula 1 below;
L is a linker containing an ethylene glycol repeating unit;
a is 0 or a natural number, with the proviso that when a is 2 or more, each L is independent of each other;
Z is an immunoglobulin Fc fragment; and
   - represents a covalent bond: 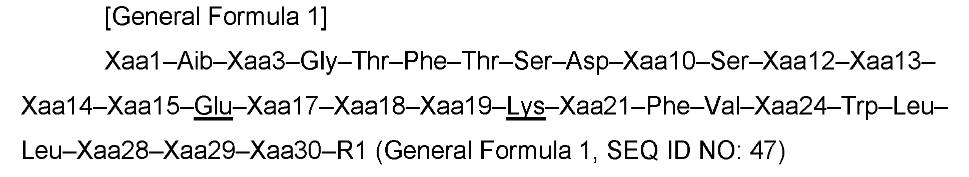
in General Formula 1 above,
a lactam ring is formed between the underlined glutamic acid (Glu) at position 16 and the underlined lysine (Lys) residue at position 20 from the N-terminus,
Xaa1 is histidine, 4-imidazoacetyl (CA), or tyrosine;
Xaa3 is glutamic acid or glutamine;
Xaa10 is tyrosine or cysteine;
Xaa12 is lysine or isoleucine;
Xaa13 is tyrosine, alanine, or cysteine;
Xaa14 is leucine or methionine;
Xaa15 is cysteine or aspartic acid;
Xaa17 is arginine, isoleucine, cysteine, or lysine;
Xaa18 is alanine, arginine, or histidine;
Xaa19 is alanine, glutamine, or cysteine;
Xaa21 is glutamic acid, or aspartic acid;
Xaa24 is glutamine, asparagine, or aspartic acid;
Xaa28 is alanine, asparagine, or aspartic acid;
Xaa29 is cysteine, glycine, glutamine, threonine, glutamic acid, or histidine;
Xaa30 is cysteine, glycine, lysine, or histidine, or is absent; and
R1 is cysteine, m-Ser-Ser-Gly-Ala-Pro-Pro-Pro-Ser-n (SEQ ID NO: 48), or m-Ser-Ser-Gly-Gln-Pro-Pro-Pro-Ser-n (SEQ ID NO: 49), or is absent,
wherein:
   m is Cys, or Pro; and
   n is Cys, or Gly, or is absent. The Aib refers to aminoisobutyric acid.

In the liquid formulation according to any one of the preceding embodiments, the liquid formulation is a liquid formulation which is characterized in that it includes: 18 nmol/mL to 920 nmol/mL of the long-acting conjugate of Chemical Formula 1 below; a buffering agent in an amount for maintaining the pH of the liquid formulation in the range of 5.0 to 7.0; and 0.5% (w/v) to 10% (w/v) of a sugar alcohol, saccharide, or a combination thereof.

In the liquid formulation according to any one of the preceding embodiments, the liquid formulation is characterized in that it may further include one or more components selected from the group consisting of an isotonic agent, a nonionic surfactant and an amino acid.

In the liquid formulation according to any one of the preceding embodiments, the liquid formulation is characterized in that it does not include an isotonic agent.

In the liquid formulation according to any one of the preceding embodiments, the liquid formulation is characterized in that it does not further include one or more components selected from the group consisting of a nonionic surfactant and an amino acid.

In the liquid formulation according to any one of the preceding embodiments, the peptide is characterized in that it includes any one amino acid sequence selected from SEQ ID NOS: 1 to 46.

In the liquid formulation according to any one of the preceding embodiments, the peptide is characterized in that it includes any one amino acid sequence selected from SEQ ID NOS: 1, 2, 9, 19, 21 to 27, 30 to 32, or 40 to 46.

In the liquid formulation according to any one of the preceding embodiments, the peptide is characterized in that it includes any one amino acid sequence selected from SEQ ID NO: 9, 30 to 32, or 42 to 46.

In the liquid formulation according to any one of the preceding embodiments, the peptide is characterized in that it further includes the amino acid sequence of SEQ ID NO: 9.

In the liquid formulation according to any one of the preceding embodiments, the R1 is characterized in that it is cysteine, Cys-Ser-Ser-Gly-Gln-Pro-Pro-Pro-Ser (SEQ ID NO: 50), Pro-Ser-Ser-Gly-Ala-Pro-Pro-Pro-Ser (SEQ ID NO: 51), Pro-Ser-Ser-Gly-Ala-Pro-Pro-Pro-Ser-Gly (SEQ ID NO: 52), Pro-Ser-Ser-Gly-Gln-Pro-Pro-Pro-Ser (SEQ ID NO: 53), or Pro-Ser-Ser-Gly-Gln-Pro-Pro-Pro-Ser-Cys (SEQ ID NO: 54), or is absent.

In the liquid formulation according to any one of the preceding embodiments, the L is characterized in that it is polyethylene glycol.

In the liquid formulation according to any one of the preceding embodiments, the formula weight of the ethylene glycol repeating unit moiety in L is characterized in that it is in the range of 1 kDa to 100 kDa.

In the liquid formulation according to any one of the preceding embodiments, the structure of Chemical Formula 1 above is characterized in that it is represented by the structure of Chemical Formula 2 below:

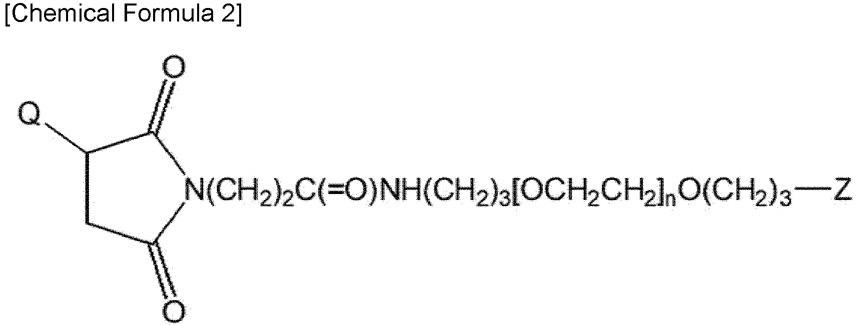

Here, Q and Z are as defined in Chemical Formula 1.

In the liquid formulation according to any one of the preceding embodiments, the ethylene glycol repeating unit is characterized in that it has a formula of [OCH₂CH₂]ₙ, wherein n is a natural number, and the average molecular weight of the [OCH₂CH₂]ₙ region in the peptide, for example, the number average molecular weight is determined to be 1 kDa to 100 kDa.

In the liquid formulation according to any one of the preceding embodiments, the value of n is characterized in that it is determined such that the average molecular weight of the [OCH₂CH₂]ₙ region in the peptide, for example, the number average molecular weight, is 10 kDa.

In the liquid formulation according to any one of the preceding embodiments, the Q is characterized in that it is amidated at the C-terminus thereof.

In the liquid formulation according to any one of the preceding embodiments, the Q is characterized in that it is linked via a sulfur atom of cysteine in the peptide.

In the liquid formulation according to any one of the preceding embodiments, the immunoglobulin Fc fragment is characterized in that it is derived from IgG4.

In the liquid formulation according to any one of the preceding embodiments, the Z is characterized in that it has a structure in which two polypeptide chains are linked by an inter-disulfide bond, and are linked only through a nitrogen atom in one of the two chains.

In the liquid formulation according to any one of the preceding embodiments, the Z is characterized in that it includes a monomer of the amino acid sequence of SEQ ID NO: 76.

In the liquid formulation according to any one of the preceding embodiments, the Z is characterized in that it is a homodimer of the monomers of the amino acid sequence of SEQ ID NO: 76.

In the liquid formulation according to any one of the preceding embodiments, the Z is characterized in that it is linked through a nitrogen atom of proline at the N-terminus thereof.

In the liquid formulation according to any one of the preceding embodiments, the Z, which is an immunoglobulin Fc fragment, and Q are characterized in that they are not glycosylated.

In the liquid formulation according to any one of the preceding embodiments, the buffering agent is characterized in that it is selected from the group consisting of citric acid and a salt thereof, acetic acid and a salt thereof, histidine and a salt thereof, phosphoric acid and a salt thereof, and a combination thereof.

In the liquid formulation according to any one of the preceding embodiments, the buffering agent is characterized in that it is selected from the group consisting of a citrate buffer solution, an acetate buffer solution, a histidine buffer solution, and a combination thereof.

In the liquid formulation according to any one of the preceding embodiments, the buffering agent is characterized in that it is acetic acid and a salt thereof.

In the liquid formulation according to any one of the preceding embodiments, the pH of the liquid formulation is characterized in that it is 5.0 to 5.5.

In the liquid formulation according to any one of the preceding embodiments, the pH of the liquid formulation is characterized in that it is 5.0 to 6.5.

In the liquid formulation according to any one of the preceding embodiments, the pH of the liquid formulation is characterized in that it is 5.1 to 6.0.

In the liquid formulation according to any one of the preceding embodiments, the pH of the liquid formulation is characterized in that it is 5.1 to 5.5.

In the liquid formulation according to any one of the preceding embodiments, the concentration of the buffering agent is characterized in that it is 5 mM to 100 mM for maintaining the pH of the liquid formulation in the range of 5.0 to 7.0.

In the liquid formulation according to any one of the preceding embodiments, the saccharide or sugar alcohol is characterized in that it is one or more selected from the group consisting of sucrose, mannitol, and sorbitol.

In the liquid formulation according to any one of the preceding embodiments, the saccharide or sugar alcohol is characterized in that it is present in a concentration of 1% (w/v) to 20% (w/v) in the formulation.

In the liquid formulation according to any one of the preceding embodiments, the long-acting conjugate is characterized in that it is present in a concentration of 18 nmol/mL to 2,757 nmol/mL, 18 nmol/mL to 920 nmol/mL, 18 nmol/mL to 919 nmol/mL, 90 nmol/mL to 552 nmol/mL, 150 nmol/mL to 600 nmol/mL, 183 nmol/mL to 552 nmol/mL, 400 nmol/mL to 550 nmol/mL, or 150 nmol/mL to 200 nmol/mL in the formulation.

In the liquid formulation according to any one of the preceding embodiments, the saccharide is characterized in that it is glucose, fructose, galactose, lactose, maltose, sucrose, or a combination thereof.

In the liquid formulation according to any one of the preceding embodiments, the saccharide is characterized in that it is sucrose.

In the liquid formulation according to any one of the preceding embodiments, the sugar alcohol is characterized in that it is one or more selected from the group consisting of mannitol and sorbitol.

In the liquid formulation according to any one of the preceding embodiments, the liquid formulation is characterized in that it further includes one or more components selected from the group consisting of a nonionic surfactant and an amino acid.

In the liquid formulation according to any one of the preceding embodiments, the liquid formulation is characterized in that it further includes an isotonic agent.

In the liquid formulation according to any one of the preceding embodiments, the isotonic agent is characterized in that it is sodium chloride.

In the liquid formulation according to any one of the preceding embodiments, the nonionic surfactant is characterized in that it is contained at a concentration of 0.01% (w/v) to 0.1% (w/v).

In the liquid formulation according to any one of the preceding embodiments, the nonionic surfactant is characterized in that it is poloxamer, polysorbate, or a combination thereof.

In the liquid formulation according to any one of the preceding embodiments, the nonionic surfactant is characterized in that it is selected from the group consisting of poloxamer 188, polysorbate 20, polysorbate 40, polysorbate 60, polysorbate 80, and a combination thereof.

In the liquid formulation according to any one of the preceding embodiments, the liquid formulation is characterized in that it further includes an amino acid selected from the group consisting of arginine, glycine, methionine, and a combination thereof as a stabilizer.

In the liquid formulation according to any one of the preceding embodiments, the liquid formulation is characterized in that it includes:
90 nmol/mL to 552 nmol/mL of a peptide conjugate of Chemical Formula 1;
5 mM to 25 mM of a buffering agent selected from citric acid and a salt thereof, acetic acid and a salt thereof, histidine and a salt thereof, phosphoric acid and a salt thereof, and a combination thereof so that the pH of the liquid formulation is 5.0 to 6.5;
1% (w/v) to 10% (w/v) of a sugar alcohol, saccharide, or a combination thereof;
0.01% (w/v) to 0.1% (w/v) of a nonionic surfactant selected from poloxamer, polysorbate, or a combination thereof; and
0.01 mg/mL to 1 mg/mL of a stabilizer selected from the group consisting of arginine, glycine, methionine, and a combination thereof.

In the liquid formulation according to any one of the preceding embodiments, the liquid formulation is characterized in that it includes:
90 nmol/mL to 552 nmol/mL of a peptide conjugate of Chemical Formula 1;
5 mM to 25 mM of a buffering agent selected from citric acid and a salt thereof, acetic acid and a salt thereof, histidine and a salt thereof, phosphoric acid and a salt thereof, and a combination thereof so that the pH of the liquid formulation is 5.0 to 5.5;
4% (w/v) to 10% (w/v) of a saccharide;
0.01% (w/v) to 0.1% (w/v) of a nonionic surfactant selected from poloxamer, polysorbate, or a combination thereof; and
0.01 mg/mL to 1 mg/mL of a stabilizer selected from the group consisting of arginine, glycine, methionine, and a combination thereof.

In the liquid formulation according to any one of the preceding embodiments, the liquid formulation is characterized in that it includes:
90 nmol/mL to 552 nmol/mL of a peptide conjugate of Chemical Formula 1;
5 mM to 25 mM of a buffering agent selected from citric acid and a salt thereof, acetic acid and a salt thereof, histidine and a salt thereof, phosphoric acid and a salt thereof, and a combination thereof so that the pH of the liquid formulation is 5.1 to 5.5;
4% (w/v) to 10% (w/v) of a saccharide; and
0.01 mg/mL to 1 mg/mL of a stabilizer selected from the group consisting of arginine, glycine, methionine, and a combination thereof.

In the liquid formulation according to any one of the preceding embodiments, the liquid formulation is characterized in that it includes:
90 nmol/mL to 552 nmol/mL of a peptide conjugate of Chemical Formula 1;
5 mM to 25 mM of a buffering agent selected from citric acid and a salt thereof, acetic acid and a salt thereof, histidine and a salt thereof, phosphoric acid and a salt thereof, and a combination thereof so that the pH of the liquid formulation is 5.1 to 5.5; and
4% (w/v) to 10% (w/v) of a saccharide.

In the liquid formulation according to any one of the preceding embodiments, the liquid formulation is characterized in that it includes:
90 nmol/mL to 552 nmol/mL of a peptide conjugate of Chemical Formula 1;
5 mM to 25 mM of a buffering agent selected from citric acid and a salt thereof, acetic acid and a salt thereof, histidine and a salt thereof, phosphoric acid and a salt thereof, and a combination thereof so that the pH of the liquid formulation is 5.0 to 5.5; and
4% (w/v) to 10% (w/v) of a saccharide.

In the liquid formulation according to any one of the preceding embodiments, the liquid formulation is characterized in that it includes:
90 nmol/mL to 552 nmol/mL of a peptide conjugate of Chemical Formula 1;
5 mM to 25 mM of a buffering agent selected from citric acid and a salt thereof, acetic acid and a salt thereof, histidine and a salt thereof, phosphoric acid and a salt thereof, and a combination thereof so that the pH of the liquid formulation is 5.0 to 5.5;
4% (w/v) to 10% (w/v) of a saccharide; and
0.01 mg/mL to 1 mg/mL of a stabilizer selected from the group consisting of arginine, glycine, methionine, and a combination thereof.

In the liquid formulation according to any one of the preceding embodiments, the liquid formulation is characterized in that it includes:
90 nmol/mL to 552 nmol/mL of a peptide conjugate of Chemical Formula 1;
5 mM to 25 mM of a buffering agent selected from citric acid and a salt thereof, acetic acid and a salt thereof, histidine and a salt thereof, phosphoric acid and a salt thereof, and a combination thereof so that the pH of the liquid formulation is 5.0 to 5.5;
4% (w/v) to 10% (w/v) of a saccharide; and
0.01% (w/v) to 0.1% (w/v) of a nonionic surfactant selected from poloxamer, polysorbate, or a combination thereof; and

In the liquid formulation according to any one of the preceding embodiments, the liquid formulation is characterized in that it includes:
90 nmol/mL to 552 nmol/mL of a peptide conjugate of Chemical Formula 1;
5 mM to 25 mM of a buffering agent selected from citric acid and a salt thereof, acetic acid and a salt thereof, histidine and a salt thereof, phosphoric acid and a salt thereof, and a combination thereof so that the pH of the liquid formulation is 5.0 to 5.5;
4% (w/v) to 10% (w/v) of a saccharide;
0.01 mg/mL to 1 mg/mL of a stabilizer selected from the group consisting of arginine, glycine, methionine, and a combination thereof; and
0.01% (w/v) to 0.1% (w/v) of a nonionic surfactant selected from poloxamer, polysorbate, or a combination thereof.

In the liquid formulation according to any one of the preceding embodiments, the amino acid is characterized in that it is methionine.

In the liquid formulation according to any one of the preceding embodiments, the methionine is characterized in that it is present in a concentration of 0.01 mg/mL to 1 mg/mL in the formulation.

In the liquid formulation according to any one of the preceding embodiments, the amino acid is characterized in that it is methionine or arginine.

In the liquid formulation according to any one of the preceding embodiments, the methionine or arginine is characterized in that it is present in a concentration of 0.01 mg/mL to 1 mg/mL in the formulation.

In the liquid formulation according to any one of the preceding embodiments, the liquid formulation is characterized in that it has a transparent appearance when stored for one week under harsh test conditions of 40°C ± 2°C and relative humidity of 75% ± 5%

In the liquid formulation according to any one of the preceding embodiments, the immunoglobulin Fc region is characterized in that it is an IgG-, IgA-, IgD-, IgE-, or IgM-derived Fc fragment.

In the liquid formulation according to any one of the preceding embodiments, the immunoglobulin Fc region is characterized in that it is selected from the group consisting of (a) CH1 domain, CH2 domain, CH3 domain, and CH4 domain; (b) CH1 domain and CH2 domain; (c) CH1 domain and CH3 domain; (d) CH2 domain and CH3 domain; (e) a combination between one or two or more domains among CH1 domain, CH2 domain, CH3 domain and CH4 domain, and an immunoglobulin hinge region or a part of the hinge region), and (f) a dimer between each domain of the heavy chain constant region and the light chain constant region.

In the liquid formulation according to any one of the preceding embodiments, each domain of the immunoglobulin Fc fragment is characterized in that it is a hybrid of domains having different origins derived from an immunoglobulin selected from the group consisting of IgG, IgA, IgD, IgE, and IgM.

In the liquid formulation according to any one of the preceding embodiments, the immunoglobulin Fc fragment is characterized in that it is in the form of a dimer or multimer, composed of a single-chain immunoglobulin consisting of domains of the same origin.

In the liquid formulation according to any one of the preceding embodiments, the immunoglobulin Fc fragment is characterized in that it is an IgG4 Fc fragment.

In the liquid formulation according to any one of the preceding embodiments, the immunoglobulin Fc fragment is characterized in that it is a human aglycosylated IgG4 Fc fragment.

In the liquid formulation according to any one of the preceding embodiments, the immunoglobulin Fc fragment is characterized in that it is a native Fc derivative, including: a modification where the site capable of forming an inter-disulfide bond is removed; a modification where several N-terminal amino acids from native Fc are removed; a modification where a methionine residue is added to the N-terminus of native Fc; a modification where complement binding sites are removed; a modification where antibody-dependent cell-mediated cytotoxicity (ADCC) sites are removed, or a combination thereof.

Another aspect for implementing the present invention provides a method for preparing the liquid formulation.

In one embodiment, the present invention relates to a method for preparing a liquid formulation of a long-acting conjugate, which is the liquid formulation according to any one of the preceding embodiments, including: mixing (a) a long-acting conjugate of a peptide having activities for a glucagon receptor, a GLP-1 receptor and a GIP receptor, in which the peptide having activities for a glucagon receptor, a GLP-1 receptor and a GIP receptor, and an immunoglobulin Fc fragment are linked to each other, with (b) i) a buffering agent and ii) a saccharide or sugar alcohol.

In the method according to any one of the preceding embodiments, the liquid formulation is characterized in that it further includes one or more components selected from the group consisting of an isotonic agent, a nonionic surfactant and an amino acid.

### [Mode for Carrying Out the Invention]

Hereinafter, the present invention will be described in more detail.

Meanwhile, each of the explanations and exemplary embodiments disclosed herein can be applied to each other explanation and exemplary embodiment. That is, all of the combinations of various factors disclosed herein belong to the scope of the present invention. Moreover, the scope of the present invention should not be limited by the specific disclosure provided hereinbelow.

Additionally, those of ordinary skill in the art may be able to recognize or confirm, using only conventional experimentation, many equivalents to the particular aspects of the invention described herein. Furthermore, it is also intended that these equivalents be included in the present invention.

Throughout the entire specification of the present invention, not only the conventional one-letter and three-letter codes for naturally occurring amino acids, but also those three-letter codes generally allowed for other amino acids, such as α-aminoisobutyric acid (Aib), Sar (N-methylglycine), and α-methyl-glutamic acid, *etc.* are used. Additionally, the amino acids mentioned herein are abbreviated according to the nomenclature rules of IUPAC-IUB as follows:

| | |
|---|---|
| alanine Ala, A | arginine Arg, R |
| asparagine Asn, N | aspartic acid Asp, D |
| cysteine Cys, C | glutamic acid Glu, E |
| glutamine Gln, Q | glycine Gly, G |
| histidine His, H | isoleucine Ile, I |
| leucine Leu, L | lysine Lys, K |
| methionine Met, M | phenylalanine Phe, F |
| proline Pro, P | serine Ser, S |
| threonine Thr, T | tryptophan Trp, W |
| tyrosine Tyr, Y | valine Val, V |

One aspect for implementing the present invention provides a liquid formulation of a long-acting conjugate of glucagon, GLP-1 (Glucagon-like peptide-1), and GIP (Glucose-dependent insulinotropic polypeptide) trigonal agonist.

Specifically, the present invention relates to a liquid formulation of a long-acting conjugate of a peptide having activities for a glucagon receptor, a GLP-1 receptor, and a GIP receptor, including: a long-acting conjugate of a peptide having activities for a glucagon receptor, a GLP-1 receptor, and a GIP receptor and a peptide having activities for a glucagon receptor, a GLP-1 receptor, and a GIP receptor, in which immunoglobulin Fc fragments are linked, in a pharmaceutically effective amount; and a buffering agent, and sugar alcohol, saccharide or a combination thereof.

Specifically, the present invention provides a liquid formulation including: a long-acting conjugate of Chemical Formula 1 below; a buffering agent; and sugar alcohol, saccharide or a combination thereof:

[Chemical Formula 1] Q - Lₐ - Z

In Chemical Formula 1 above,
Q is a peptide of General Formula 1 below;
L is a linker containing an ethylene glycol repeating unit;
a is 0 or a natural number, with the proviso that when a is 2 or more, each L is independent of each other;
Z is an immunoglobulin Fc fragment; and
   - represents a covalent bond: 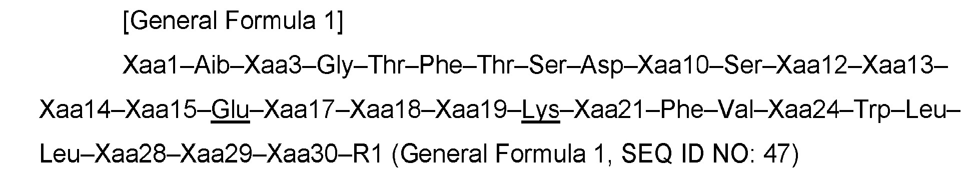
in General Formula 1 above,
a lactam ring is formed between the underlined glutamic acid (Glu) at position 16 and the underlined lysine (Lys) residue at position 20 from the N-terminus, wherein
Xaa1 is histidine, 4-imidazoacetyl (CA), or tyrosine;
Xaa3 is glutamic acid or glutamine;
Xaa10 is tyrosine or cysteine;
Xaa12 is lysine or isoleucine;
Xaa13 is tyrosine, alanine, or cysteine;
Xaa14 is leucine or methionine;
Xaa15 is cysteine or aspartic acid;
Xaa17 is arginine, isoleucine, cysteine, or lysine;
Xaa18 is alanine, arginine, or histidine;
Xaa19 is alanine, glutamine, or cysteine;
Xaa21 is glutamic acid, or aspartic acid;
Xaa24 is glutamine, asparagine, or aspartic acid;
Xaa28 is alanine, asparagine, or aspartic acid;
Xaa29 is cysteine, glycine, glutamine, threonine, glutamic acid, or histidine;
Xaa30 is cysteine, glycine, lysine, or histidine, or is absent; and
R1 is cysteine, m-Ser-Ser-Gly-Ala-Pro-Pro-Pro-Ser-n (SEQ ID NO: 48), or m-Ser-Ser-Gly-Gln-Pro-Pro-Pro-Ser-n (SEQ ID NO: 49), or is absent,
wherein:
   m is Cys, or Pro; and
   n is Cys, or Gly, or is absent.

As used herein, the term "liquid formulation" refers to a drug formulated into a liquid form and is intended to include all liquid formulations for internal use and formulations for external use.

The liquid formulation of the present invention includes a long-acting conjugate of Chemical Formula 1 showing a pharmacological effect, and a substance capable of stably maintaining and/or storing the conjugate showing the pharmacological effect for a certain period of time when it is formulated in a liquid form. Components included in addition to the long-acting conjugate of Chemical Formula 1 that exhibit the pharmacological effect of the liquid formulation may be used interchangeably with a stabilizer.

In the liquid formulation of the long-acting conjugate of Chemical Formula 1 of the present invention, storage stability is important for ensuring accurate dosage.

It was confirmed that the long-acting conjugate of Chemical Formula 1 is stable even when stored for a long time by including a specific concentration of the long-acting conjugate of Chemical Formula 1, which is a substance exhibiting a pharmacological effect; an amount of buffering agent to maintain the pH in the range of 5.0 to 7.0; and 0.5% (w/v) to 10% (w/v) of a sugar alcohol, saccharide, or a combination thereof, thereby providing a novel formulation of the present invention.

The concentration of the long-acting conjugate of a peptide having activities for a glucagon receptor, a GLP-1 receptor, and a GIP receptor included in the liquid formulation of the present invention may be 18 nmol/mL to 1,840 nmol/mL, but is not limited thereto.

In one embodiment, the concentration of the long-acting conjugate may be 18 nmol/mL to 920 nmol/mL, but is not limited thereto.

As used herein, the term "stabilizer" refers to a substance that stably maintains components such as active ingredients for a specific period of time in a formulation. The stabilizer of the present invention preferably contains no albumin. Human serum albumin that can be used as a protein stabilizer is prepared from human blood, and thus can be contaminated with human pathogenic virus, and gelatin or bovine serum albumin can cause diseases or can cause allergic reactions in some patients. The albumin-free stabilizer of the present invention does not contain a foreign protein such as human or animal serum albumin or purified gelatin, and thus is not susceptible to viral infection.

In the present invention, the stabilizer particularly refers to a substance that allows the long-acting conjugate of glucagon, GLP-1, and GIP trigonal agonist to be stored stably. In the long-acting conjugate of glucagon, GLP-1, and GIP trigonal agonist, the storage stability is not only important to ensure an accurate, but also to inhibit potential generation of antigenic substances against the conjugate of glucagon, GLP-1, and GIP trigonal agonist.

The buffering agent, which is one component included in the liquid formulation of the present invention, can maintain the pH of a solution so that the pH of the liquid formulation does not rapidly change so as to make the long-acting conjugate of Chemical Formula 1 stable. The buffering agent may also be referred to as a buffer system, and the buffering agent or buffer system serves to maintain the pH of the liquid formulation. Any buffering agent capable of maintaining a pH that can stabilize the long-acting conjugate of Chemical Formula 1, which is the target material for stabilization, may be used without limitation.

The buffering agent may be a pH buffer, including phosphoric acid and its conjugate base(i.e., alkali salt such as phosphate: sodium phosphate, potassium phosphate, or a hydrogen or dihydrogen salt thereof), citric acid and a salt thereof (e.g., sodium citrate), acetic acid and a salt thereof (e.g., sodium acetate), or histidine and a salt thereof, and a mixture of these buffers may also be used, but the buffering agent is not limited thereto.

The liquid formulation of the present invention may include a buffer solution containing the buffering agent as a solvent of the liquid formulation, specifically, the buffer solution may be selected from the group consisting of a citrate buffer solution (e.g., a sodium citrate buffer solution), an acetate buffer solution (e.g., a sodium acetate buffer solution), a phosphate buffer solution (e.g., a sodium phosphate buffer solution), a histidine buffer solution, and a combination thereof. Additionally, the buffer solution or the buffering agent in the liquid formulation (citric acid and a salt thereof, acetic acid and a salt thereof, histidine and a salt thereof, phosphoric acid and a salt thereof, or a combination thereof) may be contained in a concentration sufficient to maintain a target pH of the liquid formulation.

The pH of the liquid formulation may be in the range of about 5.0 to about 7.0, for example, about 5.0 to about 6.8, about 5.0 to about 6.7, about 5.0 to about 6.6, about 5.0 to about 6.5, about 5.0 to about 6.4, about 5.0 to about 6.3, about 5.0 to about 6.2, about 5.0 to about 6.1, about 5.0 to 6.0, about 5.0 to about 5.9, about 5.0 to 5.8, about 5.0 to about 5.7, about 5.0 to about 5.6, about 5.0 to about 5.5, about 5.0 to about 5.4, about 5.0 to about 5.3, about 5.0 to about 5.2, about 5.1 to about 7.0, for example, about 5.1 to about 6.8, about 5.1 to about 6.7, about 5.1 to about 6.6, about 5.1 to about 6.5, about 5.1 to about 6.4, about 5.1 to about 6.3, about 5.1 to about 6.2, about 5.1 to about 6.1, about 5.1 to 6.0, about 5.1 to about 5.9, about 5.1 to 5.8, about 5.1 to about 5.7, about 5.1 to about 5.6, about 5.1 to about 5.5, about 5.1 to about 5.4, about 5.1 to about 5.3, about 5.1 to about 5.2, or a about 5.0, 5.1, 5.2, 5.3, 5.4, 5.5, 5.6, 5.7, 5.8, 5.9, 6.0, 6.1, 6.2, 6.3, 6.4, 6.5, 6.6, 6.7, 6.8, 6.9, or 7.0, but is not particularly limited thereto.

The concentration of the liquid formulation to reach the target pH may be about 1 mM to about 200 mM, more specifically, about 5 mM to about 100 mM, about 5 mM to about 80 mM, about 5 mM to about 40 mM, about 8 mM to about 40 mM, about 5 mM to about 30 mM, or about 5 mM to about 25 mM, about 10 mM to about 25 mM, about 15 mM to about 25 mM, about 18 mM to about 24 mM, about 18 mM to about 22 mM, or about 20 mM, but is not particularly limited thereto.

In one embodiment, the buffering agent may be acetic acid and a salt thereof, but is not limited thereto.

In another embodiment, the buffer solution may be an acetate buffer solution (*e.g*., sodium acetate buffer solution) or a citrate buffer solution (*e.g*., sodium citrate buffer solution), but is not particularly limited thereto.

Meanwhile, in the preparation of the liquid formulation, the components are dissolved in water (*e.g*., WFI), and the pH of the buffer solution or formulation can be adjusted to a desired pH using HCl and/or NaOH, *etc.,* which is a method already commonly used in the art. Therefore, even if the pH adjuster is not additionally mentioned in the claims, it will be understood by those skilled in the art that the formulation can have an adjusted pH through such a method.

The sugar alcohol, which is one component included in the stabilizer of the present invention, refers to a substance containing a plurality of hydroxyl groups, and may include a substance, in which an aldehyde group and/or a ketone group of a saccharide is substituted with an alcohol group, and saccharides containing multiple hydroxyl groups. The saccharide or sugar alcohol may increase the stability of the long-acting conjugate of glucagon, GLP-1, and GIP trigonal agonist. For example, the sugar alcohol may be one or more selected from the group consisting of mannitol and sorbitol, but is not limited thereto.

The saccharide, which is one component included in the liquid formulation of the present invention, refers to monosaccharides, disaccharides, polysaccharides, oligosaccharides, *etc.,* and can increase the stability of the long-acting conjugate of the peptide having activities for a glucagon receptor, a GLP-1 receptor, and a GIP receptor. Specific examples may include monosaccharides such as mannose, glucose, fructose, galactose, fucose, and xylose; disaccharides such as lactose, maltose, and sucrose; and polysaccharides such as raffinose and dextran, but are not limited thereto.

In one embodiment, the saccharide may be glucose, fructose, galactose, lactose, maltose, sucrose, or a combination thereof, but is not limited thereto.

For example, the saccharide may be sucrose, but is not particularly limited thereto.

The sugar alcohol, saccharide, or a combination thereof may be present in a concentration of about 0.5% (w/v) to about 20% (w/v), about 0.5% (w/v) to about 10% (w/v), about 0.5% (w/v) to about 6% (w/v), about 1% (w/v) to about 20% (w/v), about 1% (w/v) to 15% (w/v), about 2% (w/v) to about 15% (w/v), about 2% (w/v) to about 12% (w/v), about 2% (w/v) to about 12% (w/v), about 3% (w/v) to about 10% (w/v), about 4% (w/v) to about 10% (w/v), about 4% (w/v) to about 6% (w/v),about 5% (w/v) to about 10% (w/v), about 6% (w/v) to about 10% (w/v), about 7% (w/v) to about 10% (w/v), about 7% (w/v) to about 9% (w/v), about 8% (w/v) to about 9% (w/v), or about 1.0% (w/v), about 3.0% (w/v), about 5.0% (w/v), or about 8.0% (w/v) relative to the total solution of the liquid formulation, but is not particularly limited thereto.

Additionally, the liquid formulation may further include one or more components selected from the group consisting of an isotonic agent, a nonionic surfactant and an amino acid, but is not particularly limited thereto.

Accordingly, the stabilizer of the liquid formulation may consist essentially of i) a buffering agent and ii) a saccharide or sugar alcohol, but may consist essentially of i) a buffering agent, ii) a saccharide or sugar alcohol, and iii) a nonionic surfactant; i) a buffering agent, ii) a saccharide or sugar alcohol, and iii) an isotonic agent; i) a buffering agent, ii) a saccharide or sugar alcohol, iii) an amino acid; and iv) a nonionic surfactant; i) a buffering agent, ii) a saccharide or sugar alcohol, and iii) an amino acid; i) a buffering agent, ii) a saccharide or sugar alcohol, iii) a nonionic surfactant, and iv) an isotonic agent; i) a buffering agent, ii) a saccharide or sugar alcohol, iii) a nonionic surfactant, and iv) an amino acid; i) a buffering agent, ii) a saccharide or sugar alcohol, iii) an isotonic agent, and iv) an amino acid; or i) a buffering agent, ii) a saccharide or sugar alcohol, iii) a nonionic surfactant, iv) an isotonic agent, and v) an amino acid, but is not particularly limited thereto. Here, it is apparent that all of the contents described above or below apply to the type, concentration, or pH of each component constituting the stabilizer.

Although not particularly limited thereto, the nonionic surfactant, which is one component included in the liquid formulation, lowers the surface tension of the protein solution, thereby preventing protein adsorption or aggregation on the hydrophobic surface.

Specific examples of the nonionic surfactant that can be used in the present invention may include polysorbates (*e.g*., polysorbate 20 (polyoxyethylene (20) sorbitan monolaurate), polysorbate 40 (polyoxyethylene (20) sorbitan monopalmitate), polysorbate 60 (polyoxyethylene (20) sorbitan monostearate), polysorbate 80 (polyoxyethylene (20) sorbitan monooleate); the numerical value 20 after the polyoxyethylene group means the total number of oxyethylene groups - (CH₂CH₂O)-), poloxamer (PEO-PPO-PEO copolymer; PEO: polyethylene oxide), PPO: polypropylene oxide)), polyethylene-polypropylene glycol, polyoxyethylene compounds (*e.g*., polyoxyethylene-stearate, polyoxyethylene alkyl ethers (alkyl: C₁-C₃₀), polyoxyethylene monoallyl ether, alkylphenyl polyoxyethylene copolymer (alkyl: C₁-C₃₀), *etc.),* sodium dodecyl sulfate (SDS), *etc.,* or polysorbate or poloxamer. These may also be used alone or in a combination of two or more thereof.

Specifically, the nonionic surfactant may be polysorbate 80, polysorbate 60, polysorbate 40, polysorbate 20, or poloxamer 188, and these may be used in combination, but is not particularly limited thereto.

In the present invention, it is preferable that the nonionic surfactant is not contained in a high concentration, and specifically, it may be contained in a concentration of about 0.2% (w/v) or less, for example, about 0.001% (w/v) to about 0.2% (w/v), about 0.001% (w/v) to about 0.1% (w/v), about 0.001% (w/v) to about 0.05% (w/v), about 0.005% (w/v) to about 0.08% (w/v), about 0.002% (w/v) to about 0.05% (w/v), about 0.005% (w/v) to about 0.05% (w/v), about 0.01% (w/v) to about 0.05% (w/v), about 0.01% (w/v) to about 0.04% (w/v), about 0.01% (w/v) to about 0.03% (w/v), about 0.01% (w/v) to about 0.1% (w/v), or about 0.02% (w/v) in the formulation of the present invention, but the concentration is not particularly limited thereto.

The amino acid, which is a type of stabilizer as an optional component that may be added to the liquid formulation, may be methionine, arginine, glycine, or a combination thereof, but is not limited thereto. In addition, the amino acid may be in the L-form, but is not particularly limited thereto. The amino acid may be methionine or arginine. Further, the methionine may be L-methionine, and the arginine may be L-arginine, but is not particularly limited thereto.

The amino acid may inhibit the generation of impurities that may occur due to the oxidation reaction of the protein, but is not particularly limited thereto.

The amino acid may be present in a concentration of about 0.01 mg/mL to about 1 mg/mL, about 0.01 mg/mL to about 0.8 mg/mL, about 0.01 mg/mL to about 0.5 mg/mL, about 0.02 mg/mL to about 0.5 mg/mL, or about 0.02 mg/mL to about 0.4 mg/mL, or about 0.1 mg/mL in the formulation, but is not particularly limited thereto.

In one embodiment, the liquid formulation including a buffering agent, and a saccharide or sugar alcohol may or may not include an isotonic agent, and may not further include one or more components selected from the group consisting of a nonionic surfactant and an amino acid, but is not limited thereto.

The isotonic agent refers to a substance that can control osmotic pressure. The isotonic agent may serve to properly maintain osmotic pressure when the liquid formulation according to the present invention is administered to the body.

Representative examples of the isotonic agent may include sodium chloride, sodium sulfate, or sodium citrate as a water-soluble inorganic salt, specifically sodium chloride, but is not particularly limited thereto. Such an inorganic salt may be an optional component further included in the above-described stabilizer, and is not particularly limited thereto. Additionally, the above-described stabilizer may serve as an isotonic agent.

The concentration of the isotonic agent in the formulation according to the present invention may be 0 mM to about 200 mM, 0 mM to about 150 mM, 0 mM to about 100 mM, about 10 mM to about 200 mM, about 10 mM to about 150 mM, about 10 mM to about 100 mM, about 10 mM to about 50 mM, about 20 mM to about 100 mM, about 40 mM to about 110 mM, about 20 mM to about 80 mM, about 20 mM to about 50 mM, about 20 mM to about 30 mM, or about 40 mM to about 50 mM, about 40 mM to about 60 mM, or about 90 mM to about 110 mM, but is not particularly limited thereto.

Meanwhile, the liquid formulation of the present invention may optionally further include other components or materials known in the art within the range that does not impair the effects of the present invention, in addition to sugar alcohol, saccharide, or a combination thereof; and a buffering agent, which are essential components of the above-described liquid formulation; and a nonionic surfactant and an amino acid, which are optional components, but is not limited thereto.

Meanwhile, the liquid formulation may further include polyhydric alcohol, but is not particularly limited thereto.

For example, the liquid formulation may include i) a buffering agent and ii) a saccharide or sugar alcohol as well as polyhydric alcohol, and may further include polyhydric alcohol in the stabilizer consisting essentially of i) a buffering agent, ii) a saccharide or sugar alcohol, and iii) a nonionic surfactant; i) a buffering agent, ii) a saccharide or sugar alcohol, and iii) an isotonic agent; i) a buffering agent, ii) a saccharide or sugar alcohol, iii) an amino acid; and iv) a nonionic surfactant; i) a buffering agent, ii) a saccharide or sugar alcohol, and iii) an amino acid; i) a buffering agent, ii) a saccharide or sugar alcohol, iii) a nonionic surfactant, and iv) an isotonic agent; i) a buffering agent, ii) a saccharide or sugar alcohol, iii) a nonionic surfactant, and iv) an amino acid; i) a buffering agent, ii) a saccharide or sugar alcohol, iii) an isotonic agent, and iv) an amino acid; or i) a buffering agent, ii) a saccharide or sugar alcohol, iii) a nonionic surfactant, iv) an isotonic agent, and v) an amino acid, but is not particularly limited thereto.

Examples of polyhydric alcohols that may be further included in the liquid formulation of the present invention may include propylene glycol and low-molecular weight polyethylene glycol, glycerol, low-molecular weight polypropylene glycol, *etc.,* and these may be used in one or two or more combinations thereof, but are not limited thereto.

In the present invention, the long-acting conjugate of Chemical Formula 1 is an active ingredient included in the liquid formulation of the present invention, and may be included therein in a pharmaceutically effective amount. For example, the concentration of the long-acting conjugate in the formulation may be about 18 nmol/mL to about 2,800 nmol/mL, about 18 nmol/mL to about 2,757 nmol/mL, about 18 nmol/mL to about 2,576 nmol/mL, about 18 nmol/mL to about 2,392 nmol/mL, about 18 nmol/mL to about 2,208 nmol/mL, about 18 nmol/mL to about 2,024 nmol/mL, about 18 nmol/mL to about 1,840 nmol/mL, about 18 nmol/mL to about 1,656 nmol/mL, about 18 nmol/mL to about 1,472 nmol/mL, about 18 nmol/mL to about 1,288 nmol/mL, about 18 nmol/mL to about 1,104 nmol/mL, about 18 nmol/mL to about 920 nmol/mL, about 18 nmol/mL to about 919 nmol/mL, about 18 nmol/mL to about 828 nmol/mL, about 18 nmol/mL to about 740 nmol/mL, about 18 nmol/mL to about 736 nmol/mL, about 18 nmol/mL to about 644 nmol/mL, about 18 nmol/mL to about 552 nmol/mL, about 18 nmol/mL to about 460 nmol/mL, about 18 nmol/mL to about 368 nmol/mL, about 18 nmol/mL to about 276 nmol/mL, about 18 nmol/mL to about 184 nmol/mL, about 18 nmol/mL to about 92 nmol/mL, about 90 nmol/mL, about 552 nmol/mL, about 90 nmol/mL, about 600 nmol/mL, about 450 nmol/mL, about 600 nmol/mL, about 500 nmol/mL, about 600 nmol/mL, about 530 nmol/mL, about 580 nmol/mL, about 183 nmol/mL, about 184 nmol/mL, about 183.79 nmol/mL, about 183.8 nmol/mL, about 183 nmol/mL to about 276 nmol/mL, about 183 nmol/mL to about 386 nmol/mL, about 183 nmol/mL to about 460 nmol/mL, about 183 nmol/mL to about 552 nmol/mL, about 183 nmol/mL to about 560 nmol/mL, about 183 nmol/mL to about 644 nmol/mL, about 183 nmol/mL to about 736 nmol/mL, about 183 nmol/mL to about 740 nmol/mL, about 551 nmol/mL, about 552 nmol/mL, about 551.37 nmol/mL, about 551.4 nmol/mL, about 150 nmol/mL to about 200 nmol/mL, about 170 nmol/mL to about 200 nmol/mL, but is not particularly limited thereto.

As used herein, the term "about" refers to a range which includes all of ±0.5, ±0.4, ±0.3, ±0.2, ±0.1, *etc.* and includes all of the values that are equivalent or similar to those following the values, but the range is not limited thereto.

In one embodiment, the liquid formulation may be a liquid formulation containing a peptide conjugate of Chemical Formula 1; a buffering agent selected from citric acid and a salt thereof, acetic acid and a salt thereof, histidine and a salt thereof, phosphoric acid and a salt thereof, and a combination thereof so that the pH of the liquid formulation is 5.0 to 5.5; a saccharide; a nonionic surfactant selected from poloxamer, polysorbate, or a combination thereof; and a stabilizer selected from the group consisting of arginine, glycine, methionine, and a combination thereof.

In one embodiment, the liquid formulation may be a liquid formulation containing 90 nmol/mL to 552 nmol/mL of a peptide conjugate of Chemical Formula 1; 5 mM to 25 mM of a buffering agent selected from citric acid and a salt thereof, acetic acid and a salt thereof, histidine and a salt thereof, phosphoric acid and a salt thereof, and a combination thereof so that the pH of the liquid formulation is 5.0 to 6.5; 1% (w/v) to 10% (w/v) of a sugar alcohol, saccharide, or a combination thereof; 0.01% (w/v) to 0.1% (w/v) of a nonionic surfactant selected from poloxamer, polysorbate, or a combination thereof; and 0.01 mg/mL to 1 mg/mL of a stabilizer selected from the group consisting of arginine, glycine, methionine, and a combination thereof.

In one embodiment, the liquid formulation may be a liquid formulation containing 90 nmol/mL to 552 nmol/mL of a peptide conjugate of Chemical Formula 1; 5 mM to 25 mM of a buffering agent selected from citric acid and a salt thereof, acetic acid and a salt thereof, histidine and a salt thereof, phosphoric acid and a salt thereof, and a combination thereof so that the pH of the liquid formulation is 5.0 to 5.5; 4% (w/v) to 10% (w/v) of a saccharide; 0.01% (w/v) to 0.1% (w/v) of a nonionic surfactant selected from poloxamer, polysorbate, or a combination thereof; and 0.01 mg/mL to 1 mg/mL of a stabilizer selected from the group consisting of arginine, glycine, methionine, and a combination thereof.

In one embodiment, the liquid formulation may be a liquid formulation containing 90 nmol/mL to 552 nmol/mL of a peptide conjugate of Chemical Formula 1; 5 mM to 25 mM of a buffering agent selected from citric acid and a salt thereof, acetic acid and a salt thereof, histidine and a salt thereof, phosphoric acid and a salt thereof, and a combination thereof so that the pH of the liquid formulation is 5.1 to 5.5; 4% (w/v) to 10% (w/v) of a saccharide; and 0.01 mg/mL to 1 mg/mL of a stabilizer selected from the group consisting of arginine, glycine, methionine, and a combination thereof.

In one embodiment, the liquid formulation may be a liquid formulation containing 90 nmol/mL to 552 nmol/mL of a peptide conjugate of Chemical Formula 1; 5 mM to 25 mM of a buffering agent selected from citric acid and a salt thereof, acetic acid and a salt thereof, histidine and a salt thereof, phosphoric acid and a salt thereof, and a combination thereof so that the pH of the liquid formulation is 5.1 to 5.5; and 4% (w/v) to 10% (w/v) of a saccharide.

In one embodiment, the liquid formulation may be a liquid formulation containing 90 nmol/mL to 552 nmol/mL of a peptide conjugate of Chemical Formula 1; 5 mM to 25 mM of a buffering agent selected from citric acid and a salt thereof, acetic acid and a salt thereof, histidine and a salt thereof, phosphoric acid and a salt thereof, and a combination thereof so that the pH of the liquid formulation is 5.1 to 5.5; and 4% (w/v) to 10% (w/v) of a saccharide.

In one embodiment, the liquid formulation may be a liquid formulation containing 90 nmol/mL to 552 nmol/mL of a peptide conjugate of Chemical Formula 1; 5 mM to 25 mM of a buffering agent selected from citric acid and a salt thereof, acetic acid and a salt thereof, histidine and a salt thereof, phosphoric acid and a salt thereof, and a combination thereof so that the pH of the liquid formulation is 5.0 to 5.5; and 4% (w/v) to 10% (w/v) of a saccharide.

In one embodiment, the liquid formulation may be a liquid formulation containing 90 nmol/mL to 552 nmol/mL of a peptide conjugate of Chemical Formula 1; 5 mM to 25 mM of a buffering agent selected from citric acid and a salt thereof, acetic acid and a salt thereof, histidine and a salt thereof, phosphoric acid and a salt thereof, and a combination thereof so that the pH of the liquid formulation is 5.0 to 5.5; 4% (w/v) to 10% (w/v) of a saccharide; and 0.01 mg/mL to 1 mg/mL of a stabilizer selected from the group consisting of arginine, glycine, methionine, and a combination thereof.

In one embodiment, the liquid formulation may be a liquid formulation containing 90 nmol/mL to 552 nmol/mL of a peptide conjugate of Chemical Formula 1; 5 mM to 25 mM of a buffering agent selected from citric acid and a salt thereof, acetic acid and a salt thereof, histidine and a salt thereof, phosphoric acid and a salt thereof, and a combination thereof so that the pH of the liquid formulation is 5.0 to 5.5; 4% (w/v) to 10% (w/v) of a saccharide; and 0.01% (w/v) to 0.1% (w/v) of a nonionic surfactant selected from poloxamer, polysorbate, or a combination thereof.

In one embodiment, the liquid formulation may be a liquid formulation containing 90 nmol/mL to 552 nmol/mL of a peptide conjugate of Chemical Formula 1; 5 mM to 25 mM of a buffering agent selected from citric acid and a salt thereof, acetic acid and a salt thereof, histidine and a salt thereof, phosphoric acid and a salt thereof, and a combination thereof so that the pH of the liquid formulation is 5.0 to 5.5; 4% (w/v) to 10% (w/v) of a saccharide; 0.01 mg/mL to 1 mg/mL of a stabilizer selected from the group consisting of arginine, glycine, methionine, and a combination thereof; and 0.01% (w/v) to 0.1% (w/v) of a nonionic surfactant selected from poloxamer, polysorbate, or a combination thereof.

The liquid formulation may have a transparent appearance when stored for one week under harsh test conditions of 40°C ± 2°C and relative humidity of 75% ± 5%.

As used herein, the term "stress testing" refers to a test intended to identify the fundamental characteristics of the stability of a drug. It is carried out during drug development and is conducted under harsher conditions than accelerated tests, and it helps to identify expected degradation products and physical changes of the drug.

Meanwhile, hereinafter, the long-acting conjugate of a peptide having activities for a glucagon receptor, a GLP-1 receptor, and a GIP receptor, which are active ingredients included in the liquid formulation of the present invention, will be described in more detail.

As used herein, the term "long-acting conjugate of a peptide having activities for a glucagon receptor, a GLP-1 receptor, and a GIP receptor" is an active ingredient included in the liquid formulation of the present invention, and may be included in the formulation in a pharmacologically effective amount. The long-acting conjugate may be in a form in which an immunoglobulin Fc fragment is conjugated to a peptide having activities for a glucagon receptor, a GLP-1 receptor, and a GIP receptor.

The conjugate may exhibit an increased duration of efficacy compared to a peptide, to which an immunoglobulin is not conjugated, and in the present invention, the conjugate according to Chemical Formula 1 of the peptide of General Formula 1 is referred to as a "long-acting conjugate", and may be used interchangeably with a "long-acting trigonal agonist conjugate", "peptide conjugate" or "long-acting conjugate of Chemical Formula 1".

In one embodiment, the immunoglobulin Fc fragment and Q may not be glycosylated, but is not limited thereto.

Meanwhile, the conjugate may be non-naturally occurring.

The long-acting conjugate of the present invention may be in a form in which the peptide having activities for a glucagon receptor, a GLP-1 receptor and a GIP receptor, and an immunoglobulin Fc fragment are linked to each other, and the linking method is not particularly limited thereto, but the peptide and the immunoglobulin Fc fragment may be linked to each other through a linker.

In one embodiment, the long-acting conjugate of the present invention has the structure of Chemical Formula 1 below.

[Chemical Formula 1] Q - Lₐ - Z

In Chemical Formula 1 above,
Q is a peptide of General Formula 1 below;
L is a linker containing an ethylene glycol repeating unit;
a is 0 or a natural number, with the proviso that when a is 2 or more, each L is independent of each other;
Z is an immunoglobulin Fc fragment; and
   - represents a covalent bond.

The Q of the long-acting conjugate of Chemical Formula 1 may be a peptide having activities for a glucagon receptor, a GLP-1 receptor and a GIP receptor. The "peptide having activities for a glucagon receptor, a GLP-1 receptor and a GIP receptor" include various substances having a significant level of activities for a glucagon receptor, a GLP-1 receptor and a GIP receptor, for example, various peptides.

Although it is not particularly limited thereto, the peptide having a significant level of activities for a glucagon receptor, a GLP-1 receptor and a GIP receptor, may be used interchangeably with a "trigonal agonist".

More specifically, the Q of Chemical Formula 1 above, which is a peptide having activities for a glucagon receptor, a GLP-1 receptor, and a GIP receptor, may a peptide having activities for a glucagon receptor, a GLP-1 receptor, and a GIP receptor, including the sequence of General Formula q below: 
[General Formula 1]
Xaa1–Aib–Xaa3–Gly–Thr–Phe–Thr–Ser–Asp–Xaa10–Ser–Xaa12–Xaa13–
Xaa14–Xaa15–Glu–Xaa17–Xaa18–Xaa19–Lys–Xaa21–Phe–Val–Xaa24–Trp–Leu–
Leu–Xaa28–Xaa29–Xaa30–R1 (General Formula 1, SEQ ID NO: 47)
 in General Formula 1 above,
a lactam ring is formed between the underlined glutamic acid (Glu) at position 16 and the underlined lysine (Lys) residue at position 20 from the N-terminus, wherein
Xaa1 is histidine, 4-imidazoacetyl (CA), or tyrosine;
Xaa3 is glutamic acid or glutamine;
Xaa10 is tyrosine or cysteine;
Xaa12 is lysine or isoleucine;
Xaa13 is tyrosine, alanine, or cysteine;
Xaa14 is leucine or methionine;
Xaa15 is cysteine or aspartic acid;
Xaa17 is arginine, isoleucine, cysteine, or lysine;
Xaa18 is alanine, arginine, or histidine;
Xaa19 is alanine, glutamine, or cysteine;
Xaa21 is glutamic acid, or aspartic acid;
Xaa24 is glutamine, asparagine, or aspartic acid;
Xaa28 is alanine, asparagine, or aspartic acid;
Xaa29 is cysteine, glycine, glutamine, threonine, glutamic acid, or histidine;
Xaa30 is cysteine, glycine, lysine, or histidine, or is absent; and
R1 is cysteine, m-Ser-Ser-Gly-Ala-Pro-Pro-Pro-Ser-n (SEQ ID NO: 48), or m-Ser-Ser-Gly-Gln-Pro-Pro-Pro-Ser-n (SEQ ID NO: 49), or is absent,
wherein:
   m is Cys, or Pro; and
   n is Cys, or Gly, or is absent.

The Aib of General Formula 1 above refers to aminoisobutyric acid.

In the present specification, the "Aib" may be used interchangeably with "2-aminoisobutyric acid" or "aminoisobutyric acid", and 2-aminoisobutyric acid and aminoisobutyric acid may be used interchangeably with each other.

The peptide may include an amino acid sequence selected from SEQ ID NOS: 1 to 46 or consist (essentially) of an amino acid sequence selected from the group consisting of SEQ ID NOS: 1 to 46, but is not limited thereto.

Examples of such a peptide include a peptide including or consisting (essentially) of an amino acid sequence selected from the group consisting of SEQ ID NOS: 1, 2, 9, 19, 21 to 27, 30 to 32, or 40 to 46, but are not particularly limited thereto. In another example, the peptide may be a peptide including or consisting (essentially) of an amino acid sequence selected from the group consisting of SEQ ID NO: 9, 30 to 32, or 42 to 46, or it may be a peptide including or consisting (essentially) of the amino acid sequence of SEQ ID NO: 9, but is not particularly limited thereto.

Additionally, the R1 in General Formula 1 above may be Cys-Ser-Ser-Gly-Gln-Pro-Pro-Pro-Ser (SEQ ID NO: 50), Pro-Ser-Ser-Gly-Ala-Pro-Pro-Pro-Ser (SEQ ID NO: 51), Pro-Ser-Ser-Gly-Ala-Pro-Pro-Pro-Ser-Gly (SEQ ID NO: 52), Pro-Ser-Ser-Gly-Gln-Pro-Pro-Pro-Ser (SEQ ID NO: 53), or Pro-Ser-Ser-Gly-Gln-Pro-Pro-Pro-Ser-Cys (SEQ ID NO: 54), or may be absent, but is no particularly limited thereto.

Additionally, the peptide having activities for a glucagon receptor, a GLP-1 receptor, and a GIP receptor may include an intramolecular bridge, *e.g.,* a covalent bridge or a non-covalent bridge, and specifically, may be in a form including a ring. It may be in a form where a ring is formed between the glutamic acid at position 16 and the lysine residue at position 20, which are underlined in General Formula 1 above, but is not particularly limited thereto. Non-limiting examples of the ring may include a lactam bridge (or lactam ring).

Further, the peptide according to the present invention may include all of those in the form of the peptide itself, a salt thereof (*e.g*., a pharmaceutically acceptable salt of the peptide), or a solvate thereof. Additionally, the peptide may be in any pharmaceutically acceptable form.

The kind of the salt is not particularly limited. However, the salt is preferably one that is safe and effective to a subject (*e.g*., a mammal), but is not particularly limited thereto.

The term "pharmaceutically acceptable" refers to a material which can be effectively used for the intended use within the scope of pharmaco-medical decision without inducing excessive toxicity, irritation, allergic responses, *etc.*

As used herein, the term "pharmaceutically acceptable salt" refers to a salt derived from pharmaceutically acceptable inorganic acids, organic acids, or bases. Examples of the suitable salts may include hydrochloric acid, bromic acid, sulfuric acid, nitric acid, perchloric acid, fumaric acid, maleic acid, phosphoric acid, glycolic acid, lactic acid, salicylic acid, succinic acid, toluene-p-sulfonic acid, tartaric acid, acetic acid, citric acid, methanesulfonic acid, formic acid, benzoic acid, malonic acid, naphthalene-2-sulfonic acid, benzenesulfonic acid, *etc.* Examples of the salts derived from suitable bases may include alkali metals such as sodium, potassium, *etc*.; alkali earth metals such as magnesium; ammonium, *etc.*

As used herein, the term "solvate" refers to a complex formed between the peptide according to the present invention or a salt thereof and a solvent molecule.

Additionally, although described as "a peptide consisting of a particular SEQ ID NO" in the present invention, it does not exclude a meaningless sequence upstream or downstream of the amino acid sequence of the corresponding SEQ ID NO, or a mutation that may occur naturally, or a silent mutation thereof, as long as the peptide has an activity the same as or corresponding to that of the peptide which consists of an amino acid sequence of the corresponding SEQ ID NO, and even when the sequence addition or mutation is present, it obviously belongs to the scope of the present invention.

Meanwhile, the peptide may be non-naturally occurring.

The C-terminus of the peptide may be an amidated peptide or a peptide having a free carboxyl group (-COOH), or may include a peptide having an unmodified C-terminus, but is not limited thereto.

In one embodiment, the Q may be amidated at the C-terminus, but is not limited thereto.

In one embodiment, the Q may be non-glycosylated, but is not limited thereto.

The peptide of General Formula 1 of the present invention can be synthesized through a solid phase synthesis method, can be produced by a recombinant method, and can be produced by commercially, but is not limited thereto.

As used herein, the term "long-acting conjugate of Chemical Formula 1", being an active ingredient included in the liquid formulation of the present invention, may be included in the liquid formulation in a pharmacologically effective amount. Specifically, the conjugate is in a form, in which the peptide having activities for a glucagon receptor, a GLP-1 receptor and a GIP receptor, and an immunoglobulin Fc region are linked to each other by a linker, and may exhibit an enhanced duration of efficacy compared to a peptide having activities for a glucagon receptor, a GLP-1 receptor and a GIP receptor to which an immunoglobulin Fc region is not linked.

Additionally, in the long-acting conjugate of Chemical Formula 1, the linkage between Q, the peptide having activities for a glucagon receptor, a GLP-1 receptor and a GIP receptor, and the immunoglobulin Fc fragment may be achieved by a physical or chemical bond, a non-covalent or covalent bond, and specifically, a covalent bond, but is not limited thereto.

Additionally, in the peptide conjugate of Chemical Formula 1, the method of linking Q, the peptide having activities for a glucagon receptor, a GLP-1 receptor and a GIP receptor, and the immunoglobulin Fc fragment is not particularly limited, but the peptide having activities for a glucagon receptor, a GLP-1 receptor and a GIP receptor, and the immunoglobulin Fc fragment may be linked to each other through a linker.

Specifically, the long-acting conjugate included in the liquid formulation of the present invention may be one represented by Chemical Formula 1 above.

In Chemical Formula 1 above, Q and Z may be linked to each other through L by a covalent bond.

More specifically, Q and L, and L and Z may be linked to each other by a covalent bond, and in particular, the conjugate may be a conjugate in which Q, L, and Z are each linked by a covalent bond in the order of Chemical Formula 1.

Additionally, Q may be directly linked to Z (*i.e*., a is 0 in Chemical Formula 1 above) or may be linked through a linker (L).

In one embodiment, Lₐ, which is one component of the long-acting conjugate of Chemical Formula 1, may be a linker containing an ethylene glycol repeating unit, *e.g*., (polyethylene glycol), and additionally, those derivatives which are already known in the art and the derivatives that can easily be prepared at the technological level of those skilled in the art are included in the scope of the present invention.

The L, which is a linker containing an ethylene glycol repeating unit, may include a functional group at the end, which is used in the preparation of the conjugate, before it is formed into a conjugate. The long-acting conjugate according to the present invention may be in the form in which Q and Z are linked through the functional group, but is not limited thereto. In the present invention, the linker containing an ethylene glycol repeating unit may include two, or three or more functional groups, and each functional group may be the same as or different from each other, but is not limited thereto.

Specifically, the linker may be polyethylene glycol (PEG) represented by Chemical Formula 3 below, but is not limited thereto: 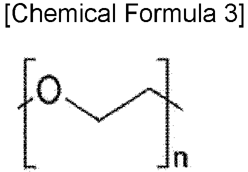 wherein n is 10 to 2,400, n is 10 to 480, or n is 50 to 250, but the range of n is not limited thereto.

In the long-acting conjugate above, the PEG moiety may include not only the -(CH₂CH₂O)ₙ- structure, but also an oxygen atom interposed between a linking element and the -(CH₂CH₂O)ₙ- structure, but the PEG moiety is not limited thereto.

The polyethylene glycol is a general term including all of the forms of homopolymers of ethylene glycol, PEG copolymers, and monomethyl-substituted PEG polymers (mPEG), but the is not particularly limited thereto.

In one embodiment, the ethylene glycol repeating unit may be represented by, for example, [OCH₂CH₂]ₙ, and the value of n is a natural number and the average molecular weight of the [OCH₂CH₂]ₙ region in the peptide conjugate, for example, the number average molecular weight, may be determined to be greater than 0 to about 100 kDa, but is not limited thereto. In another example, the value of n is a natural number, and the average molecular weight of the [OCH₂CH₂]ₙ region in the peptide conjugate, for example, the number average molecular weight, may be about 1 kDa to about 100 kDa, about 1 kDa to about 80 kDa, about 1 kDa to about 50 kDa, about 1 kDa to about 30 kDa, about 1 kDa to about 25 kDa, about 1 kDa to about 20 kDa, about 1 kDa to about 15 kDa, about 1 kDa to about 13 kDa, about 1 kDa to about 11 kDa, about 1 kDa to about 10 kDa, about 1 kDa to about 8 kDa, about 1 kDa to about 5 kDa, about 1 kDa to about 3.4 kDa, about 3 kDa to about 30 kDa, about 3 kDa to about 27 kDa, about 3 kDa to about 25 kDa, about 3 kDa to about 22 kDa, about 3 kDa to about 20 kDa, about 3 kDa to about 18 kDa, about 3 kDa to about 16 kDa, about 3 kDa to about 15 kDa, about 3 kDa to about 13 kDa, about 3 kDa to about 11 kDa, about 3 kDa to about 10 kDa, about 3 kDa to about 8 kDa, about 3 kDa to about 5 kDa, about 3 kDa to about 3.4 kDa, about 8 kDa to about 30 kDa, about 8 kDa to about 27 kDa, about 8 kDa to about 25 kDa, about 8 kDa to about 22 kDa, about 8 kDa to about 20 kDa, about 8 kDa to about 18 kDa, about 8 kDa to about 16 kDa, about 8 kDa to about 15 kDa, about 8 kDa to about 13 kDa, about 8 kDa to about 11 kDa, about 8 kDa to about 10 kDa, about 9 kDa to about 15 kDa, about 9 kDa to about 14 kDa, about 9 kDa to about 13 kDa, about 9 kDa to about 12 kDa, about 9 kDa to about 11 kDa, about 9.5 kDa to about 10.5 kDa, or about 10 kDa, but is not limited thereto.

In one embodiment, both ends of the linker may be bound to a thiol group, an amino group, or a hydroxyl group of the immunoglobulin Fc region, and may be bound to a thiol group, an amino group, an azide group, or a hydroxyl group of the peptide of General Formula 1.

Specifically, the linker may include a reactive group capable of binding to each of the immunoglobulin Fc and the peptide of General Formula 1 at both ends. Specifically, the linker may include a reactive group that can bind to a thiol group of cysteine; an amino group located at the N-terminus, lysine, arginine, glutamine, and/or histidine; and/or a hydroxyl group located at the C-terminus in the immunoglobulin Fc fragment, and that can bind to a thiol group of cysteine; an amino group of lysine, arginine, glutamine, and/or histidine; an azide group of azido-lysine; and/or a hydroxyl group in the peptide of General Formula 1, but the reactive groups are not limited thereto.

More specifically, the reactive group of the linker may be one or more selected from the group consisting of an aldehyde group, a maleimide group, and a succinimide derivative, but is not limited thereto.

In the above, as an example of the aldehyde group, a propionaldehyde group or butyraldehyde group may be used, but the aldehyde group is not limited thereto.

In the above, as a succinimide derivative, succinimidyl carboxymethyl, succinimidyl valerate, succinimidyl methylbutanoate, succinimidyl methylpropionate, succinimidyl butanoate, succinimidyl propionate, *N*-hydroxysuccinimide, hydroxy succinimidyl, or succinimidyl carbonate may be used, but the succinimide derivative is not limited thereto.

The linker may be linked to Z, the immunoglobulin Fc, and Q, the peptide of General Formula 1, through the reactive groups to be converted to a linker moiety.

Additionally, the final product produced through reductive amination by an aldehyde bond is much more stable than that linked by an amide bond. The aldehyde reactive group selectively reacts at the N-terminus at a low pH, while it can form a covalent bond with a lysine residue at a high pH (e.g., pH 9.0), but is not limited thereto.

The terminal reactive groups of the linker of the present invention may be the same as or different from each other. The linker may have an aldehyde reactive group at both ends. Alternatively, the linker may have an aldehyde group and a maleimide group at each end, or may have an aldehyde group and a succinimide reactive group at each end, but is not limited thereto.

For example, the linker may have a maleimide group at one end and an aldehyde group, a propionaldehyde group, or a butyraldehyde group at the other end. As another example, the linker may have a succinimidyl group at one end and a propionaldehyde group or butyraldehyde group at the other end.

When a polyethylene glycol having a hydroxy reactive group at propionaldehyde end is used as a linker, the conjugate of the present invention may be prepared by activating the hydroxy group to various reactive groups by known chemical reactions or by using a commercially available polyethylene glycol having a modified reactive group.

In a specific embodiment, the reactive group of the linker may be linked to a cysteine residue of the peptide of General Formula 1, more specifically to the -SH group of cysteine, but the linker is not limited thereto.

When maleimide-PEG-aldehyde is used, the maleimide group may be linked to the -SH group of the peptide of General Formula 1 by a thioether bond, and the aldehyde group may be linked to the -NH₂ group of the immunoglobulin Fc through reductive alkylation, but is not limited thereto, and this is merely an embodiment.

Through the reductive alkylation, a structure such as -PEG-O-CH₂CH₂CH₂NH-immunoglobulin Fc may be formed by linking an amino group at the N-terminus of an immunoglobulin Fc fragment to an oxygen atom located at one end of the PEG through a linker reactive group having a structure of -CH₂CH₂CH₂-; and a structure, in which one end of the PEG is linked to a sulfur atom located at the cysteine of the peptide of General Formula 1 through a thioether bond, may be formed. The thioether bond described above may include the following structure:

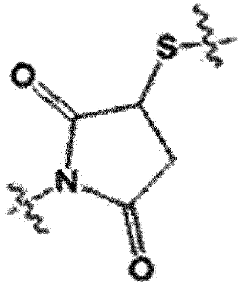

However, the linker is not particularly limited to the above embodiment, and it is merely an embodiment.

Additionally, in the above conjugate, the reactive group of the linker may be linked to -NH₂ located at the N-terminus of an immunoglobulin Fc fragment, but this is merely an embodiment.

In addition, in the conjugate, the peptide of General Formula 1 may be linked to a linker having a reactive group through the C-terminus, but this is merely an embodiment.

As used herein, the term "C-terminus" refers to a carboxy terminus of a peptide, and refers to a position capable of binding with the linker for the purpose of the present invention. For example, the C-terminus may include all of the amino acid residue at the most terminal end of the C-terminus and amino acid residues near the C-terminus, and specifically, may include the 1^{st} to 20^{th} amino acid residues from the most terminal end, although the C-terminus is not limited thereto.

In one embodiment, the conjugate of Chemical Formula 1 may have a structure of Chemical Formula 2 below:

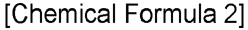

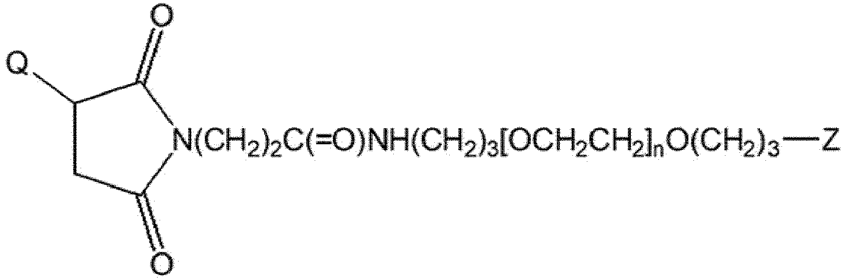

In Chemical Formula 2, Q is the peptide of Chemical Formula 1 described above;
Z is an immunoglobulin Fc fragment; and
n may be a natural number. In particular, the description of n is the same as described above.

In one embodiment, the long-acting conjugate of Chemical Formula 2 has a structure in which the peptide Q of Chemical Formula 1 of SEQ ID NO: 47 and the immunoglobulin Fc fragment Z are covalently linked through an ethylene glycol repeating unit, wherein each Q may be linked to a succinimide ring of Chemical Formula 2, and Z may be linked to an oxypropylene group of Chemical Formula 2.

In Chemical Formula 2 above, the value of n may be determined such that the average molecular weight of the [OCH₂CH₂]ₙ region in the peptide conjugate, for example, the number average molecular weight, is 1 kDa to 100 kDa, or 1 kDa to 20 kDa, or 10 kDa, but not limited thereto.

The Q of the peptide conjugate may be a peptide having activities for a glucagon receptor, a GLP-1 receptor, and a GIP receptor.

In one embodiment, the moiety at which Q is linked to the succinimide ring of Chemical Formula 2 may be a sulfur atom of the C-terminal cysteine of Q.

The Z of Chemical Formula 1 or 2 is an immunoglobulin Fc fragment, and the immunoglobulin Fc region in the present specification encompasses not only a native sequence obtained from papain digestion of an immunoglobulin, but also derivatives thereof, substituents, for example, variants, in which one or more amino acid residues in the native sequence are converted by deletion, insertion, non-conservative or conservative substitution, or a combination thereof, and thus the sequence become different from the native sequence, *etc.* The above derivatives, substituents, and variants are required to retain FcRn binding ability.

The moiety linked to the oxypropylene group in Z is not specifically limited. In one embodiment of the present invention, the moiety of Z linked to the oxypropylene group may be an N-terminal nitrogen or a nitrogen atom of a internal residue of Z (*e.g*., epsilon nitrogen of lysine). In one specific embodiment of the present invention, the moiety where Z is linked to the oxypropylene group of Chemical Formula 1 may be the N-terminal proline of Z, but is not limited thereto.

The Z may have a structure in which two polypeptide chains are linked by an inter-disulfide bond, or a structure in which two polypeptide chains are linked through a nitrogen atom in only one of the two chains, but is not limited thereto. The linkage through the nitrogen atom may be linked to the epsilon amino atom or the N-terminal amino group of lysine via reductive amination.

The reductive amination reaction refers to a reaction in which an amine group or amino group of a reactant reacts with an aldehyde of another reactant (*i.e*., a functional group capable of reductive amination) to produce an amine, and an amine bond is formed by a reduction reaction thereafter. The reductive amination reaction is a reaction of organic synthesis widely known in the art.

In one embodiment, the Z may be linked through the nitrogen atom of the N-terminal proline thereof, but is not limited thereto.

As used herein, the term "immunoglobulin Fc fragment" refers to a heavy chain constant region excluding the heavy and light chain variable regions of an immunoglobulin. Specifically, the immunoglobulin Fc fragment may include the heavy chain constant region 2 (CH2) and/or heavy chain constant region 3 (CH3) portions, and more specifically, may further include a hinge region (all or part of the hinge region).

The immunoglobulin Fc fragment may be a constitution constituting the moiety of the peptide conjugate of Chemical Formula 1 of the present invention. Specifically, it may correspond to Z in Chemical Formula 1 above.

Such an immunoglobulin Fc fragment may include a hinge region in the heavy chain constant region, but is not limited thereto.

In the present invention, the immunoglobulin Fc fragment may include a specific hinge sequence in the N-terminus.

As used herein, the term "hinge sequence" refers to a region which is located in the heavy chain and forms a dimer of immunoglobulin Fc fragments through an inter-disulfide bond.

In the present invention, the hinge sequence may be modified such that a part of the hinge sequence having the following amino acid sequence is deleted and thus there is only one cysteine residue in the sequence, but is not limited thereto:
Glu-Ser-Lys-Tyr-Gly-Pro-Pro-Cys-Pro-Ser-Cys-Pro (SEQ ID NO: 55).

The hinge sequence may be one in which the 8^{th} or 11^{th} cysteine residue in the hinge sequence of SEQ ID NO: 55 is deleted and thus only one cysteine residue is included in the sequence. The hinge sequence of the present invention may consist of 3 to 12 amino acids, including only one cysteine residue, but the hinge sequence is not limited thereto. More specifically, the hinge sequence of the present invention may have the following sequences: Glu-Ser-Lys-Tyr-Gly-Pro-Pro-Pro-Ser-Cys-Pro (SEQ ID NO: 56), Glu-Ser-Lys-Tyr-Gly-Pro-Pro-Cys-Pro-Ser-Pro (SEQ ID NO: 57), Glu-Ser-Lys-Tyr-Gly-Pro-Pro-Cys-Pro-Ser (SEQ ID NO: 58), Glu-Ser-Lys-Tyr-Gly-Pro-Pro-Cys-Pro-Pro (SEQ ID NO: 59), Lys-Tyr-Gly-Pro-Pro-Cys-Pro-Ser (SEQ ID NO: 60), Glu-Ser-Lys-Tyr-Gly-Pro-Pro-Cys (SEQ ID NO: 61), Glu-Lys-Tyr-Gly-Pro-Pro-Cys (SEQ ID NO: 62), Glu-Ser-Pro-Ser-Cys-Pro (SEQ ID NO: 63), Glu-Pro-Ser-Cys-Pro (SEQ ID NO: 64), Pro-Ser-Cys-Pro (SEQ ID NO: 65), Glu-Ser-Lys-Tyr-Gly-Pro-Pro-Ser-Cys-Pro (SEQ ID NO: 66), Lys-Tyr-Gly-Pro-Pro-Pro-Ser-Cys-Pro (SEQ ID NO: 67), Glu-Ser-Lys-Tyr-Gly-Pro-Ser-Cys-Pro (SEQ ID NO: 68), Glu-Ser-Lys-Tyr-Gly-Pro-Pro-Cys (SEQ ID NO: 69), Lys-Tyr-Gly-Pro-Pro-Cys-Pro (SEQ ID NO: 70), Glu-Ser-Lys-Pro-Ser-Cys-Pro (SEQ ID NO: 71), Glu-Ser-Pro-Ser-Cys-Pro (SEQ ID NO: 72), Glu-Pro-Ser-Cys (SEQ ID NO: 73), and Ser-Cys-Pro (SEQ ID NO: 74). More specifically, the hinge sequence may include the amino acid sequence of SEQ ID NO: 74 (Ser-Cys-Pro) or SEQ ID NO: 65 (Pro-Ser-Cys-Pro), but is not limited thereto.

The immunoglobulin Fc fragment of the present invention may be in a form in which two molecules of the immunoglobulin Fc chain form a dimer due to the presence of a hinge sequence therein, and in addition, the long-acting conjugate of Chemical Formula 1 of the present invention may be in a form in which one end of the linker is linked to one chain of the dimeric immunoglobulin Fc fragments, but the immunoglobulin Fc fragment and the conjugate are not limited thereto.

As used herein, the term "N-terminus" refers to the amino terminus of a protein or polypeptide, and it may include 1, 2, 3, 4, 5, 6, 7, 8, 9, or 10 or more amino acids from the most terminal end or the most terminal end of the amino terminus. The immunoglobulin Fc fragment of the present invention may include a hinge sequence in the N-terminus, but is not limited thereto.

Additionally, the immunoglobulin Fc fragment of the present invention may be an extended Fc fragment, which includes all or part of the heavy chain constant region 1 (CH1) and/or light chain constant region 1 (CL1) excluding the heavy chain and light chain variable regions of an immunoglobulin, as long as it has substantially the equivalent or an improved effect compared to its native type. Additionally, the immunoglobulin Fc fragment of the present invention may be a region in which some fairly long amino acid sequences corresponding to CH2 and/or CH3 are removed.

For example, the immunoglobulin Fc fragment of the present invention may be 1) a CH1 domain, a CH2 domain, a CH3 domain, and a CH4 domain; 2) a CH1 domain and a CH2 domain; 3) a CH1 domain and a CH3 domain; 4) a CH2 domain and a CH3 domain; 5) a combination of (i) one or two or more domains among a CH1 domain, a CH2 domain, a CH3 domain, and a CH4 domain and (ii) an immunoglobulin hinge region (or part of the hinge region); or 6) a dimer of each domain of the heavy chain constant region and a light chain constant region, but the immunoglobulin Fc region is not limited thereto.

Additionally, in one embodiment, the immunoglobulin Fc fragment may have a dimeric form, and one molecule of the peptide of general Formula 1 may be covalently linked to one Fc region in a dimeric form, in particular, the immunoglobulin Fc and the peptide of General Formula 1 may be covalently linked to each other through a linker containing an ethylene glycol repeating unit. Meanwhile, it is also possible that two molecules of the peptide of General Formula 1 are symmetrically linked to one Fc region in a dimeric form. In particular, the immunoglobulin Fc and the peptide of General Formula 1 may be linked to each other through a linker containing an ethylene glycol repeating unit, but is not limited to the embodiments described above.

In addition, the immunoglobulin Fc fragment of the present invention includes native amino acid sequences as well as sequence derivatives thereof. The amino acid sequence derivative means that the sequence is different from the native amino acid sequence due to deletion, insertion, non-conservative or conservative substitution, or a combination thereof in one or more amino acid residues in the native amino acid sequence.

For example, amino acid residues at positions 214 to 238, 297 to 299, 318 to 322, or 327 to 331 in IgG Fc, which are known to be important for linkage, may be used as the sites suitable for modification.

Additionally, various types of derivatives are available, for example, one where the site capable of forming an inter-disulfide bond is removed; one where several N-terminal amino acids from native Fc are removed; one where a methionine residue is added to the N-terminus of native Fc, *etc.* Additionally, complement binding sites (*e.g*., C1q binding sites) or antibody-dependent cell-mediated cytotoxicity (ADCC) sites may be removed to eliminate the effector function. The techniques for preparing the sequence derivatives of these immunoglobulin Fc fragments are disclosed in International Publication Nos. WO 97/34631, WO 96/32478, *etc.*

Amino acid substitutions in a protein or peptide that do not alter the entire activity of a molecule are well known in the art (H. Neurath, R. L. Hill, The Proteins, Academic Press, New York, 1979). The most common substitutions occur between amino acid residues of Ala/Ser, Val/Ile, Asp/Glu, Thr/Ser, Ala/Gly, Ala/Thr, Ser/Asn, Ala/Val, Ser/Gly, Thy/Phe, Ala/Pro, Lys/Arg, Asp/Asn, Leu/Ile, Leu/Val, Ala/Glu, and Asp/Gly. In some cases, amino acids may be modified by phosphorylation, sulfation, acrylation, glycosylation, methylation, farnesylation, acetylation, amidation, *etc.*

The Fc derivatives described above may be those which exhibit the same biological activity as the Fc fragment of the present invention and have an increased structural stability of the Fc fragment against heat, pH, *etc.*

Additionally, such an Fc fragment may be obtained from a native type isolated from humans or animals (*e.g*., cows, goats, pigs, mice, rabbits, hamsters, rats, guinea pigs, *etc.*) or may be recombinants or derivatives thereof obtained from transformed animal cells or microorganisms. In particular, the Fc region may be obtained from a native immunoglobulin by isolating a whole immunoglobulin from a living human or animal and treating the isolated immunoglobulin with protease. When the whole immunoglobulin is treated with papain, it is cleaved into Fab and Fc fragments, whereas when treated with pepsin, it is cleaved into pF'c and F(ab)₂ fragments. Fc or pF'c can be isolated using size-exclusion chromatography, *etc.* In a more specific embodiment, the Fc fragment may be a recombinant immunoglobulin Fc fragment where a human-derived Fc fragment is obtained from a microorganism.

Additionally, the immunoglobulin Fc fragment may be in the form of native glycans, increased or decreased glycans compared to the native type, or in a deglycosylated form. The increase, decrease, or removal of the immunoglobulin Fc glycans may be achieved by conventional methods such as a chemical method, enzymatic method, and genetic engineering method using a microorganism. In particular, the immunoglobulin Fc fragment where the glycans are removed from the Fc shows a significant decrease in binding affinity for the complement (C1q) and a decrease or removal of antibody-dependent cytotoxicity or complement-dependent cytotoxicity, and thus it does not induce unnecessary immune responses *in vivo.* In this regard, an immunoglobulin Fc fragment in a deglycosylated or aglycosylated form may be more suitable to meet the original object of the present invention as a drug carrier.

As used herein, the term "deglycosylation" refers to an Fc fragment in which glycans are removed with an enzyme, and the term "aglycosylation" refers to a non-glycosylated Fc fragment produced in prokaryotes, more specifically, *E. coli.*

Meanwhile, the immunoglobulin Fc fragment may be derived from humans or animals (*e.g*., cows, goats, pigs, mice, rabbits, hamsters, rats, guinea pigs, *etc.*), and in a more specific embodiment, it may be derived from humans.

Additionally, the immunoglobulin Fc fragment may be derived from IgG, IgA, IgD, IgE, IgM, or a combination or hybrid thereof. In a more specific embodiment, the immunoglobulin Fc fragment may be derived from IgG or IgM, which are among the most abundant proteins in human blood, and in an even more specific embodiment, it may be derived from IgG, which is known to enhance the half-lives of ligand-binding proteins. In an even yet more specific embodiment, the immunoglobulin Fc fragment may be an IgG4 Fc fragment, and in the most specific embodiment, it may be an aglycosylated Fc fragment derived from a human IgG4, but is not limited thereto.

Additionally, in a specific embodiment, the immunoglobulin Fc fragment, being a human IgG4 Fc fragment, may be in the form of a homodimer in which two monomers are linked through an inter-disulfide bond (an inter-chain form) between cysteines, which are the 3^{rd} amino acid of each monomer. In particular, each monomer of the homodimer independently has/or can have an internal disulfide bond between the cysteines at positions 35 and 95; and an internal disulfide bond between the cysteines at positions 141 and 199 (*i.e*., two internal disulfide bonds (an intra-chain form)). With respect to the number of amino acids, each monomer may consist of 221 amino acids, and the amino acids forming the homodimer may consist of a total of 442 amino acids, but the number of amino acids is not limited thereto. Specifically, the immunoglobulin Fc fragment may be one in which two monomers having the amino acid sequence of SEQ ID NO: 79 (consisting of 221 amino acids) form a homodimer through an inter-disulfide bond between cysteines, which are the 3^{rd} amino acid of each monomer, and in which the monomers of the homodimer independently form an internal disulfide bond between the cysteines at positions 35 and 95 and an internal disulfide bond between the cysteines at positions 141 and 199, but the immunoglobulin Fc fragment is not limited thereto.

The Z in Chemical Formula 1 may include a monomer of the amino acid sequence of SEQ ID NO: 76, and the Z may be a homodimer of the monomers of the amino acid sequence of SEQ ID NO: 76, but is not limited thereto.

In one example, the immunoglobulin Fc fragment may be a homodimer including the amino acid sequence of SEQ ID NO: 75 (consisting of 442 amino acids), but is not limited thereto.

Meanwhile, as used herein, the term "combination" means that polypeptides encoding single-chain immunoglobulin Fc fragments of the same origin are linked to a single-chain polypeptide of a different origin to form a dimer or multimer. That is, it is possible to prepare a dimer or multimer from two or more fragments selected from the group consisting of Fc fragments of IgG Fc, IgA Fc, IgM Fc, IgD Fc, and IgE Fc.

As used herein, the term "hybrid" means that sequences corresponding two or more immunoglobulin Fc fragments of different origins are present in a single chain of an immunoglobulin constant region. In the present invention, various hybrid forms are possible. For example, the hybrid domain may be composed of one to four domains selected from the group consisting of CH1, CH2, CH3, and CH4 of IgG Fc, IgM Fc, IgA Fc, IgE Fc, and IgD Fc, and may further include a hinge region.

Meanwhile, IgG may also be divided into the IgG1, IgG2, IgG3, and IgG4 subclasses, and the present invention may include combinations or hybrids thereof. Preferred are the IgG2 and IgG4 subclasses, and most preferred is the Fc fragment of IgG4, which rarely has effector functions such as complement-dependent cytotoxicity (CDC).

Meanwhile, the liquid formulation may be for prevention of treatment of metabolic syndrome.

As used herein, the term "prevention" refers to all activities that inhibit or delay the occurrence of metabolic syndrome by administering the above conjugate or a formulation including the conjugate, and the term "treatment" refers to all activities that improve or advantageously change the symptoms of metabolic syndrome by administering the above conjugate or a formulation including the conjugate.

As used herein, the term "administration" refers to the introduction of a particular material into a patient by any appropriate method, and the administration route of the composition is not particularly limited, but may be any conventional route that enables delivery of the composition to the target in the body (e.g., intraperitoneal administration, intravenous administration, intramuscular administration, subcutaneous administration, intradermal administration, oral administration, topical administration, intranasal administration, intrapulmonary administration, intrarectal administration, *etc.*).

Another aspect for implementing the present invention provides a method for preparing the liquid formulation.

Specifically, the preparation method may include: mixing (a) a long-acting conjugate of a peptide having activities for a glucagon receptor, a GLP-1 receptor and a GIP receptor, in which the peptide having activities for a glucagon receptor, a GLP-1 receptor and a GIP receptor, and an immunoglobulin Fc fragment are linked to each other, with (b) a stabilizer including i) a buffering agent and ii) sugar alcohol, saccharide or a combination.

Meanwhile, the stabilizer may further include one or more components selected from the group consisting of an isotonic agent, a nonionic surfactant, and an amino acid, but is not particularly limited thereto.

The long-acting binder, buffering agent, sugar alcohol, saccharide or a combination thereof, isotonic agent, nonionic surfactant, amino acid, and stabilizer are the same as described above.

Still another aspect for implementing the present invention provides a method for preventing or treating metabolic syndrome, including: administering the liquid formulation to a subject in need thereof.

The liquid formulation, metabolic syndrome, prevention, and treatment are the same as described above.

The subject refers to a subject in need of administration of the liquid formulation of the present invention, and includes, without limitation, any subject that can be treated with the liquid formulation of the present invention, and specifically includes humans or mammals including rats, livestock, *etc.*

The treatment method of the present invention may include administering the liquid formulation in a pharmaceutically effective amount. An appropriate total daily dose of the liquid formulation may be determined within the scope of correct medical judgment by a practitioner, and the liquid formulation may be administered once or several times in divided doses. However, for the purpose of the present invention, it is preferred that the specific therapeutically effective dose of the liquid formulation for any particular patient be applied differently depending on the kind and degree of responses to be achieved, specific compositions including whether other agents are occasionally used therewith, the patient's age, body weight, general health conditions, sex and diet, administration time, administration route, excretion rate of the composition, duration of treatment, various factors including drugs used in combination or simultaneously with the specific compositions, and similar factors well known in the medical field.

Yet another embodiment for implementing the present invention provides the use of the liquid formulation in the preparation of a medicament for the prevention or treatment of metabolic syndrome.

The liquid formulation, metabolic syndrome, prevention, and treatment are the same as described above.

Even another embodiment for implementing the present invention provides the use of the liquid formulation for use in the prevention or treatment of metabolic syndrome.

The liquid formulation, metabolic syndrome, prevention, and treatment are the same as described above.

Hereinafter, the present invention will be described in more detail with reference to the following Examples. However, these Examples are for illustrative purposes only and the scope of the invention is not limited by these Examples.

### Preparation Example: Preparation of Long-Acting Conjugate of Glucagon, GLP-1, and GIP Trigonal Agonist

Long-acting conjugates of glucagon, GLP-1, and GIP trigonal agonist were prepared by way of the following method.

### 1-1: Preparation of Glucagon, GLP-1, and GIP Trigonal Agonist

Trigonal agonists showing activities for all of GLP-1 receptor, GIP receptor, and glucagon receptor were prepared, and their amino acid sequences are shown in Table 1 below.

**[Table 1]**

| **SEQ ID NO:** | **Sequence** | **Information** |
|---|---|---|
| 1 | Y X Q G T F T S D Y S K Y L D E C R A K E F V Q W L L D H H P S S G Q P P P S | Ring formation |
| 2 | Y X Q G T F T S D Y S K C L D E K R A K E F V Q W L L D H H P S S G Q P P P S | Ring formation |
| 3 | H X Q G T F T S D Y S K Y L D E K A Q K E F V Q W L L D T C | Ring formation |
| 4 | H X Q G T F T S D Y S K Y L D E K A C K E F V Q W L L A Q | Ring formation |
| 5 | H X Q G T F T S D Y S K Y L D E K A C K D F V Q W L L D G G P S S G A P P P S | Ring formation |
| 6 | H X Q G T F T S D Y S K Y L D E K R Q K E F V N W L L A Q K C | Ring formation |
| 7 | H X Q G T F T S D Y S K Y L C E K R Q K E F V Q W L L N G G P S S G A P P P S G | Ring formation |
| 8 | H X Q G T F T S D Y S K Y L D E C R Q K E F V Q W L L N G G P S S G A P P P S G | Ring formation |
| 9 | Y X Q G T F T S D Y S K Y L D E K R A K E F V Q W L L D H H P S S G Q P P P S C | Ring formation |
| 10 | Y X Q G T F T S D Y S K Y L D E K R A K E F V Q W L L D H H C S S G Q P P P S | Ring formation |
| 11 | H X Q G T F T S D Y S K Y L D E K R A K E F V Q W L L D H H P S S G Q P P P S C | Ring formation |
| 12 | H X Q G T F T S D Y S K Y L D E K R Q K E F V Q W L L N T C | Ring formation |
| 13 | H X Q G T F T S D Y S K Y L D E K R Q K E F V Q W L L D T C | Ring formation |
| 14 | H X E G T F T S D Y S I A M D E I H Q K D F V N W L L A Q C | Ring formation |
| 15 | H X E G T F T S D Y S I A M D E I H Q K D F V D W L L A E C | Ring formation |
| 16 | H X Q G T F T S D Y S I A M D E I H Q K D F V N W L L A Q C | Ring formation |
| 17 | H X Q G T F T S D Y S K Y L D E K R Q K E F V N W L L A Q C | Ring formation |
| 18 | H X Q G T F T S D Y S K Y L D E K R Q K E F V Q W L L N T K C | Ring formation |
| 19 | Y X Q G T F T S D Y S K Y L D E C A A K E F V Q W L L D H H P S S G Q P P P S | Ring formation |
| 20 | H X Q G T F T S D Y S K C L D E K R A K E F V Q W L L D H H P S S G Q P P P S | Ring formation |
| 21 | Y X Q G T F T S D Y S K Y L D E C R A K D F V Q W L L D H H P S S G Q P P P S | Ring formation |
| 22 | Y X Q G T F T S D Y S K Y L D E C A A K D F V Q W L L D H H P S S G Q P P P S | Ring formation |
| 23 | Y X Q G T F T S D Y S K C L D E K A A K E F V Q W L L D H H P S S G Q P P P S | Ring formation |
| 24 | Y X Q G T F T S D Y S K C L D E R A A K E F V Q W L L D H H P S S G Q P P P S | Ring formation |
| 25 | Y X Q G T F T S D Y S K C L D E K R A K D F V Q W L L D H H P S S G Q P P P S | Ring formation |
| 26 | Y X Q G T F T S D Y S K Y L D E R A C K D F V Q W L L D H H P S S G Q P P P S | Ring formation |
| 27 | Y X Q G T F T S D C S K Y L D E R A A K D F V Q W L L D H H P S S G Q P P P S | Ring formation |
| 28 | CAX Q G T F T S D Y S K Y L D E C R A K E F V Q W L L D | Ring formation |
| | H H P S S G Q P P P S | |
| 29 | CA X Q G T F T S D Y S K C L D E K R A K E F V Q W L L D H H P S S G Q P P P S | Ring formation |
| 30 | Y X Q G T F T S D Y S K Y L D E K A A K E F V Q W L L D H H P S S G Q P P P S C | Ring formation |
| 31 | Y X Q G T F T S D Y S K Y L D E K R A K D F V Q W L L D H H P S S G Q P P P S C | Ring formation |
| 32 | Y X Q G T F T S D Y S K Y L D E K A A K D F V Q W L L D H H P S S G Q P P P S C | Ring formation |
| 33 | H X Q G T F T S D Y S K Y L D E K R Q K E F V Q W L L D T K C | Ring formation |
| 34 | H X E G T F T S D Y S I A M D E I H Q K D F V N W L L A Q K C | Ring formation |
| 35 | H X E G T F T S D Y S I A M D E I H Q K D F V D W L L A E K C | Ring formation |
| 36 | CA X E G T F T S D Y S I A M D E I H Q K D F V N W L L A Q C | Ring formation |
| 37 | CA X Q G T F T S D Y S K Y L D E K R Q K E F V N W L L A Q C | Ring formation |
| 38 | CA X E G T F T S D Y S I A M D E I H Q K D F V D W L L A E K C | Ring formation |
| 39 | CA X Q G T F T S D Y S K Y L D E K R Q K E F V N W L L A Q K C | Ring formation |
| 40 | Y X Q G T F T S D Y S K Y L D E K R A K E F V Q W L L D H C P S S G Q P P P S | Ring formation |
| 41 | Y X Q G T F T S D Y S K Y L D E K R A K E F V Q W L L D C H P S S G Q P P P S | Ring formation |
| 42 | Y X Q G T F T S D Y S K A L D E K A A K E F V N W L L D H H P S S G Q P P P S C | Ring formation |
| 43 | Y X Q G T F T S D Y S K A L D E K A A K D F V N W L L D H H P S S G Q P P P S C | Ring formation |
| 44 | Y X Q G T F T S D Y S K A L D E K A A K E F V Q W L L D Q H P S S G Q P P P S C | Ring formation |
| 45 | Y X Q G T F T S D Y S K A L D E K A A K E F V N W L L D Q H P S S G Q P P P S C | Ring formation |
| 46 | Y X Q G T F T S D Y S K A L D E K A A K D F V N W L L D Q H P S S G Q P P P S C | Ring formation |

In the sequences described in Table 1, the amino acid represented by X represents aminoisobutyric acid (Aib), which is a non-natural amino acid, and the underlined amino acids represent the formation of a ring between the underlined amino acids. Additionally, in Table 1, CA represents 4-imidazoacetyl, and Y represents tyrosine.

### 1-2: Measurement of In Vitro Activities of Trigonal Agonist

In order to measure the activities of the trigonal agonists prepared in Example 1-1, a method of measuring *in vitro* cellular activities using cell lines, where a GLP-1 receptor, a glucagon (GCG) receptor, and a GIP receptor are each transformed, was used.

Each of the cell lines above is one in which the genes for a human GLP-1 receptor, a human GCG receptor, and a human GIP receptor are transformed into Chinese hamster ovary (CHO), respectively, to be expressed therein, and is thus suitable for the measurement of the activities of GLP-1, GCG, and GIP. Accordingly, the activity for each part was measured using the respective transformed cell line.

For the measurement of the GLP-1 activity of the trigonal agonists prepared in Example 1-1, human GLP-1 was subjected to a 4-fold serial dilution from 50 nM to 0.000048 nM, and the trigonal agonists prepared in Example 1-1 were subjected to a 4-fold serial dilution from 400 nM to 0.00038 nM. The culture solution was removed from the cultured CHO cells, in which the human GLP-1 receptor was expressed, and each of the serially diluted materials was added to the CHO cells in an amount of 5 µL, respectively, and a buffer solution containing a cAMP antibody was added thereto in an amount of 5 µL and cultured at room temperature for 15 minutes. Then, a detection mix containing a cell lysis buffer was added thereto in an amount of 10 µL for the lysis of the cells and reacted at room temperature for 90 minutes. The cell lysates, after completion of the reaction, were applied to the LANCE cAMP kit (PerkinElmer, USA) to calculate the EC₅₀ value through accumulated cAMP, and the values were compared with one another. The relative titers relative to human GLP-1 are shown in Table 2 below.

For the measurement of the GCG activity of the trigonal agonists prepared in Example 1-1, human GCG was subjected to a 4-fold serial dilution from 50 nM to 0.000048 nM, and the trigonal agonists prepared in Example 1-1 were subjected to a 4-fold serial dilution from 400 nM to 0.00038 nM. The culture solution was removed from the cultured CHO cells, in which the human GCG receptor was expressed, and each of the serially diluted materials was added to the CHO cells in an amount of 5 µL, respectively, and a buffer solution containing a cAMP antibody was added thereto in an amount of 5 µL and cultured at room temperature for 15 minutes. Then, a detection mix containing a cell lysis buffer was added thereto in an amount of 10 µL for the lysis of the cells and reacted at room temperature for 90 minutes. The cell lysates, after completion of the reaction, were applied to the LANCE cAMP kit (PerkinElmer, USA) to calculate the EC₅₀ value through accumulated cAMP, and the values were compared with one another. The relative titers relative to human GCG are shown in Table 2 below.

For the measurement of the GIP activity of the trigonal agonists prepared in Example 1-1, human GIP was subjected to a 4-fold serial dilution from 50 nM to 0.000048 nM, and the trigonal agonists prepared in Example 1-1 were subjected to a 4-fold serial dilution from 400 nM to 0.00038 nM. The culture solution was removed from the cultured CHO cells, in which the human GIP receptor was expressed, and each of the serially diluted materials was added to the CHO cells in an amount of 5 µL, respectively, and a buffer solution containing a cAMP antibody was added thereto in an amount of 5 µL and cultured at room temperature for 15 minutes. Then, a detection mix containing a cell lysis buffer was added thereto in an amount of 10 µL for the lysis of the cells and reacted at room temperature for 90 minutes. The cell lysates, after completion of the reaction, were applied to the LANCE cAMP kit (PerkinElmer, USA) to calculate the EC₅₀ value through accumulated cAMP, and the values were compared with one another. The relative titers relative to human GIP are shown in Table 2 below.

**[Table 2]**

| Relative titer ratio of trigonal agonists | | | |
|---|---|---|---|
| | *In Vitro* Activity Compared to Native Peptide (%) | | |
| SEQ ID NO: | vs. GLP-1 | vs. Glucagon | vs. GIP |
| 1 | 17.8 | 267 | 22.7 |
| 2 | 20.1 | 140 | 59.7 |
| 3 | 142 | 0.7 | 0.8 |
| 4 | 135 | 2.2 | 2.4 |
| 5 | 151 | 1.7 | 8.8 |
| 6 | 19.1 | 0.92 | 0.6 |
| 7 | 37.4 | 0.39 | 0.2 |
| 8 | 236 | 6.21 | 2.2 |
| 9 | 33.9 | 205.8 | 7.8 |
| 10 | 12.6 | 88.4 | 3.70 |
| 11 | 67.4 | 50.5 | 2.7 |
| 12 | 14.4 | 2.0 | 0.1 |
| 13 | 44.1 | 7.5 | 0.3 |
| 14 | 161 | 8.4 | 1.3 |
| 15 | 30.6 | 1.4 | 0.1 |
| 16 | 27.1 | 0.7 | 2.4 |
| 17 | 57.9 | 4.9 | 0.8 |
| 18 | 39.1 | 2.6 | 0.2 |
| 19 | 64.6 | 60.1 | 92.9 |
| 20 | 95.4 | 25.2 | 11.6 |
| 21 | 15.8 | 172 | 17.2 |
| 22 | 28.5 | 46.2 | 39.8 |
| 23 | 27.9 | 8.8 | 107 |
| 24 | 24.3 | 9.6 | 62.8 |
| 25 | 15.1 | 71.3 | 64.4 |
| 26 | 90.1 | 12.7 | 94.7 |
| 27 | 11.5 | 1.0 | 1.6 |
| 28 | 22.6 | 5.4 | 3.0 |
| 29 | 12.9 | 0.9 | 1.0 |
| 30 | 35.1 | 8.5 | 18.0 |
| 31 | 10.3 | 47.6 | 11.7 |
| 32 | 38.7 | 12.2 | 35.5 |
| 33 | 51.0 | 14.0 | 0.12 |
| 34 | 41.5 | 4.9 | 1.4 |
| 35 | 8.1 | 0.0 | 0.1 |
| 36 | 47.3 | 1.3 | 0.4 |
| 37 | 28.4 | 0.4 | 0.2 |
| 38 | 31.9 | 16.8 | 0.3 |
| 39 | 5.7 | 0.3 | 0.7 |
| 40 | 34.4 | 194.8 | 5.2 |
| 41 | 10.5 | 62.8 | 2.6 |
| 42 | 28.1 | 8.2 | 47.1 |
| 43 | 20.9 | 14.9 | 57.7 |
| 44 | 42.2 | 12.7 | 118.5 |
| 45 | 23.2 | 13.9 | 40.1 |
| 46 | 23.3 | 29.5 | 58.0 |

It was confirmed that the trigonal agonists prepared above exhibited excellent effects on all of GLP-1, GIP, and glucagon receptors.

### 1-3: Preparation of Long-Acting Conjugate of Trigonal Agonists

A long-acting conjugate was prepared using the peptide of SEQ ID NO: 9 as a representative trigonal agonist. In order to prepare a linker between the trigonal agonist and the polyethylene glycol linker, maleimide-PEG-aldehyde (Japan NOF Inc.,), which is a linearly modified polyethylene glycol with a molecular weight of 10kDa, in which the hydrogens at both terminals are substituted with a 3-(3-maleimidopropionamido)propyl group and a 3-oxopropyl group (propionaldehyde group), respectively, was allowed to react with a cysteine residue of the trigonal agonist to PEGylate the trigonal agonist at the maleimide end of maleimide-PEG-aldehyde. Specifically, the trigonal agonist and the maleimide-PEG-aldehyde were reacted at a molar ratio of 1:1 to 3 at a protein concentration of 1 mg/mL to 5 mg/mL at low temperature for 0.5 to 3 hours. In particular, the reaction was performed in an environment in which 20% to 60% isopropanol was added to 50 mM Tris buffer (pH 7.5). Upon completion of the reaction, the reaction solutions were applied to SP Sepharose HP (GE Healthcare, USA) to purify the trigonal agonist which was mono-PEGylated on cysteine.

The immunoglobulin Fc fragment was prepared using the immunoglobulin Fc fragment (49.8 kDa, a homodimer in which two chains of SEQ ID NO: 76 are linked by an intra-disulfide bond) having a hinge region of the Pro-Ser-Cys-Pro sequence at the N-terminus by the method described in International Patent Publication No. WO 2007/021129.

Then, the purified mono-PEGylated trigonal agonist and the immunoglobulin Fc were reacted at a molar ratio of 1:1 to 5 at a protein concentration of 10 mg/mL to 50 mg/mL at 4°C to 8°C for 12 to 18 hours. The reaction was performed in an environment in which 10 mM to 50 mM sodium cyanoborohydride (a reducing agent) and 10% to 30% isopropanol were added to 100 mM potassium phosphate butter (pH 6.0). Upon completion of the reaction, the reaction solutions were applied to the Butyl Sepharose FF purification column (GE Healthcare, USA) and Source ISO purification column (GE Healthcare, USA) to purify the long-acting conjugate of the trigonal agonist in which the polyethylene glycol end at the aldehyde side of the mono-PEGylated trigonal agonist is linked to the nitrogen at the N-terminal proline of one of the two chains of the immunoglobulin Fc homodimer.

After the preparation, the purity analyzed by reverse-phase chromatography, size-exclusion chromatography, and ion-exchange chromatography was shown to be 95% or more.

In particular, the conjugate, in which the glucagon, GLP-1, and GIP trigonal agonist, and the immunoglobulin Fc fragment are linked through PEG, was designated as the "long-acting conjugate of glucagon, GLP-1, and GIP trigonal agonist".

### Example 1: Evaluation of Stability of Long-Acting Conjugate of Glucagon, GLP-1, and GIP Trigonal Agonist According to pH

The stability of long-acting conjugate of glucagon, GLP-1, and GIP trigonal agonist was compared under various pH based on a liquid formulation consisting of a buffering agent, mannitol as a sugar alcohol, polysorbate 20 as a surfactant, and methionine. As a comparative example, a formulation having a pH of 4.5 was used.

The long-acting conjugate of the trigonal agonist obtained in Preparation Example above was prepared as a liquid formulation using the composition shown in Table 3 (the concentration of the long-acting conjugate is 183.79 nmol/mL) and stored at 25°C for 6 weeks, and then the stability was analyzed by ion-exchange high-performance liquid chromatography (IE-HPLC) and reverse-phase-high-performance liquid chromatography (RP-HPLC).

In Table 4, IE-HPLC (%) and RP-HPLC (%) indicate the percentage of the ratio of the area% at the time of measurement divided by the initial area% during the preservation test (area%/start area%), indicating the residual ratio from the initial concentration (183.79 nmol/mL) of the long-acting conjugate of the trigonal agonist.

**[Table 3]**

| | Buffering agent | pH | Sugar alcohol | Isotonic agent | Surfactant | Other |
|---|---|---|---|---|---|---|
| Co mpa rativ e Exa mpl e | 20 mM sodium citrate | 4.5 | 5% mannitol | - | 0.02% polysorbate 20 | 0.1 mg/mL methionine |
| #1 | 20 mM sodium citrate | 5.0 | 5% mannitol | - | 0.02% polysorbate 20 | 0.1 mg/mL methionine |
| #2 | 20 mM sodium citrate | 5.5 | 5% mannitol | - | 0.02% polysorbate 20 | 0.1 mg/mL methionine |
| #3 | 20 mM sodium citrate | 6.0 | 5% mannitol | - | 0.02% polysorbate 20 | 0.1 mg/mL methionine |
| #4 | 20 mM sodium citrate | 6.5 | 5% mannitol | - | 0.02% polysorbate 20 | 0.1 mg/mL methionine |

**[Table 4]**

| | IE-HPLC (%) | | | | RP-HPLC (%) | | | |
|---|---|---|---|---|---|---|---|---|
| | Initial | 2 weeks | 4 weeks | 6 weeks | Initial | 2 weeks | 4 weeks | 6 weeks |
| Comparative Example | 100 | 93.6 | 80.2 | N/D^{*} | 100 | 95.5 | 91.6 | N/D |
| #1 | 100 | 98.0 | 95.6 | 89.9 | 100 | 99.8 | 98.0 | 95.5 |
| #2 | 100 | 96.1 | 93.1 | 83.4 | 100 | 99.6 | 96.8 | 94.0 |
| #3 | 100 | 90.1 | 80.8 | 64.9 | 100 | 98.2 | 95.4 | 91.6 |
| #4 | 100 | 74.9 | 54.8 | 33.9 | 100 | 97.8 | 94.0 | 89.9 |

| | | | | | | | | |
|---|---|---|---|---|---|---|---|---|
| * N/D: Analysis not possible due to protein precipitation | | | | | | | | |

As can be seen from the above results, Formulation (#1) having a composition of sodium citrate and pH 5.0, and Formulation (#2) having a composition of sodium citrate and pH 5.5 showed high stability at 25°C for 6 weeks. Formulation (#3) having a composition of pH 6.0 (#3) and Formulation (#4) having a composition of pH 6.5 also exhibited stability for 6 weeks. In the case of the composition of pH 4.5, which is the Comparative Example, it was confirmed that precipitation occurred at 6 weeks.

### Example 2: Evaluation of Stability of Long-Acting Conjugate of Glucagon, GLP-1, and GIP Trigonal Agonist According to Types of Saccharides or Sugar Alcohols

Examples of saccharides or sugar alcohols that may be additionally included in order to enhance storage stability of the long-acting conjugate of glucagon, GLP-1, and GIP trigonal agonist may include monosaccharides such as mannose, glucose, fucose and xylose, and polysaccharides such as lactose, maltose, sucrose, sorbitol, raffinose and dextran. Among them, the stability of the long-acting conjugate of glucagon, GLP-1, and GIP trigonal agonist according to mannitol, sucrose, and sorbitol confirmed in Example 1 was compared. In particular, the concentrations of mannitol, sucrose and sorbitol were selected in consideration of the maximum allowable range of commercially available formulations and that recommended by the licensing agency.

The long-acting conjugate of the trigonal agonist obtained above was prepared as a liquid formulation using the composition shown in Table 5 (the concentration of the long-acting conjugate is 183.79 nmol/mL) and stored at 25°C for 6 weeks, and then the stability was analyzed by ion-exchange high-performance liquid chromatography (IE-HPLC) and reverse-phase-high-performance liquid chromatography (RP-HPLC).

In Table 6, IE-HPLC (%) and RP-HPLC (%) indicate the percentage of the ratio of the area% at the time of measurement divided by the initial area% during the preservation test (area%/start area%), indicating the residual ratio from the initial concentration (183.79 nmol/mL) of the long-acting conjugate of the trigonal agonist.

**[Table 5]**

| | Buffering agent | pH | Saccharide or Sugar alcohol | Isotonic agent | Surfactant | Other |
|---|---|---|---|---|---|---|
| #1 | 20 mM sodium citrate | 5.5 | 5% mannitol | - | 0.02% polysorbate 20 | 0.1 mg/m L methionin e |
| #2 | 20 mM sodium citrate | 5.5 | 5% sorbitol | - | 0.02% polysorbate 20 | 0.1 mg/m L methionin e |
| #3 | 20 mM sodium citrate | 5.5 | 8% sucrose | - | 0.02% polysorbate 20 | 0.1 mg/m L methionin e |

**[Table 6]**

| | IE-HPLC (%) | | | | RP-HPLC (%) | | | |
|---|---|---|---|---|---|---|---|---|
| | Initial | 2 weeks | 4 weeks | 6 weeks | Initial | 2 weeks | 4 weeks | 6 weeks |
| #1 | 100 | 96.1 | 93.1 | 83.4 | 100 | 99.6 | 96.8 | 94.0 |
| #2 | 100 | 96.3 | 92.6 | 83.3 | 100 | 99.7 | 96.9 | 94.0 |
| #3 | 100 | 96.1 | 92.5 | 82.2 | 100 | 99.7 | 97.0 | 94.0 |

As can be seen from the above results, when mannitol, sorbitol, and sucrose, which are saccharides or sugar alcohols that may be included in order to increase the storage stability of the long-acting conjugate of glucagon, GLP-1, and GIP trigonal agonist, were added at concentrations of 5%, 5%, and 8%, respectively, similar stability was observed.

### Example 3: Evaluation of Stability of Long-Acting Conjugate of Glucagon, GLP-1, and GIP Trigonal Agonist According to Concentrations of Saccharides or Sugar Alcohols

The stability of long-acting conjugate of glucagon, GLP-1, and GIP trigonal agonist according to the concentrations of saccharides or sugar alcohols was compared based on the composition (sodium citrate, pH 5.5, mannitol, polysorbate 20, and methionine) of the liquid formulation confirmed in Example 1 or 2. In particular, the concentration of the isotonic agent added together was selected in consideration of the allowable range of commercially available formulations and normal plasma osmotic pressure.

The long-acting conjugate of the trigonal agonist obtained above was prepared as a liquid formulation using the composition shown in Table 7 (the concentration of the long-acting conjugate is 183.79 nmol/mL) and stored at 25°C for 6 weeks, and then the stability was analyzed by ion-exchange high-performance liquid chromatography (IE-HPLC) and reverse-phase-high-performance liquid chromatography (RP-HPLC).

In Table 8, IE-HPLC (%) and RP-HPLC (%) indicate the percentage of the ratio of the area% at the time of measurement divided by the initial area% during the preservation test (area%/start area%), indicating the residual ratio from the initial concentration (183.79 nmol/mL) of the long-acting conjugate of the trigonal agonist.

**[Table 7]**

| | Buffering agent | pH | Sugar alcohol | Isotonic agent | Surfactant | Other |
|---|---|---|---|---|---|---|
| #1 | 20 mM sodium citrate | 5.5 | 5% mannitol | - | 0.02% polysorbate 20 | 0.1 mg/mL methionine |
| #2 | 20 mM sodium citrate | 5.5 | 3% mannitol | 50 mM NaCl | 0.02% polysorbate 20 | 0.1 mg/mL methionine |
| #3 | 20 mM sodium citrate | 5.5 | 1% mannitol | 100 mM NaCl | 0.02% polysorbate 20 | 0.1 mg/mL methionine |

**[Table 8]**

| | IE-HPLC (%) | | | | RP-HPLC (%) | | | |
|---|---|---|---|---|---|---|---|---|
| | Initial | 2 weeks | 4 weeks | 6 weeks | Initial | 2 weeks | 4 weeks | 6 weeks |
| #1 | 100 | 95.3 | 88.0 | 83.9 | 100 | 98.2 | 94.5 | 90.8 |
| #2 | 100 | 96.1 | 87.6 | 84.2 | 100 | 97.9 | 94.1 | 90.6 |
| #3 | 100 | 96.3 | 87.3 | 83.9 | 100 | 97.8 | 93.8 | 88.3 |

As can be seen from the above results, when the formulation of the long-acting conjugate of glucagon, GLP-1, and GIP trigonal agonist contained sugar alcohol at concentrations of 1% to 5%, similar stability was observed.

### Example 4: Evaluation of Stability of Long-Acting Conjugate of Glucagon, GLP-1, and GIP Trigonal Agonist According to Types of Buffering agents

The stability of long-acting conjugate of glucagon, GLP-1, and GIP trigonal agonist according to the types of buffering agents was compared based on the composition (sodium citrate, pH 5.5, mannitol, polysorbate 20, and methionine) of the liquid formulation.

The long-acting conjugate of the trigonal agonist obtained above was prepared as a liquid formulation using the composition shown in Table 9 (the concentration of the long-acting conjugate is 183.79 nmol/mL) and stored at 25°C for 6 weeks, and then the stability was analyzed by ion-exchange high-performance liquid chromatography (IE-HPLC) and reverse-phase-high-performance liquid chromatography (RP-HPLC).

In Table 10, IE-HPLC (%) and RP-HPLC (%) indicate the percentage of the ratio of the area% at the time of measurement divided by the initial area% during the preservation test (area%/start area%), indicating the residual ratio from the initial concentration (183.79 nmol/mL) of the long-acting conjugate of the trigonal agonist.

**[Table 9]**

| | Buffering agent | pH | Sugar alcohol | Isotonic agent | Surfactant | Other |
|---|---|---|---|---|---|---|
| #1 | 20 mM sodium citrate | 5.5 | 5% mannitol | - | 0.02% polysorbate 20 | 0.1 mg/mL methionine |
| #2 | 20 mM sodium acetate | 5.5 | 5% mannitol | - | 0.02% polysorbate 20 | 0.1 mg/mL methionine |
| #3 | 20 mM histidine | 5.5 | 5% mannitol | - | 0.02% polysorbate 20 | 0.1 mg/mL methionine |

**[Table 10]**

| | IE-HPLC (%) | | | | RP-HPLC (%) | | | |
|---|---|---|---|---|---|---|---|---|
| | Initial | 2 weeks | 4 weeks | 6 weeks | Initial | 2 weeks | 4 weeks | 6 weeks |
| #1 | 100 | 95.3 | 88.0 | 83.9 | 100 | 98.2 | 94.5 | 90.8 |
| #2 | 100 | 96.2 | 87.0 | 83.8 | 100 | 97.8 | 94.7 | 91.0 |
| #3 | 100 | 95.6 | 83.2 | 77.8 | 100 | 97.7 | 93.9 | 88.3 |

| | | | | | | | | |
|---|---|---|---|---|---|---|---|---|
| * N/D: Analysis not possible due to protein precipitation | | | | | | | | |

As can be seen from the above results, when sodium citrate (#1) and sodium acetate (#2) were used as the buffering agent, high stability was exhibited at 25°C for 6 weeks. Additionally, it was confirmed that good stability was exhibited even when histidine (#3) was used as the buffering agent (FIGS. 1a and b).

### Example 5: Evaluation of Stability of Long-Acting Conjugate of Glucagon, GLP-1, and GIP Trigonal Agonist According to Types of Nonionic Surfactants

The stability of long-acting conjugate of glucagon, GLP-1, and GIP trigonal agonist according to the types of nonionic surfactants was compared in the liquid formulation having the composition of sodium acetate, sucrose and methionine.

In particular, the concentrations of polysorbate 20, polysorbate 80 and poloxamer 188 as nonionic surfactants were selected in consideration of commercially available formulations.

The long-acting conjugate of the trigonal agonist obtained above was prepared as a liquid formulation using the composition shown in Table 11 (the concentration of the long-acting conjugate is 183.79 nmol/mL) and stored at 25°C for 6 weeks, and then the stability was analyzed by ion-exchange high-performance liquid chromatography (IE-HPLC) and reverse-phase-high-performance liquid chromatography (RP-HPLC).

In Table 12, IE-HPLC (%) and RP-HPLC (%) indicate the percentage of the ratio of the area% at the time of measurement divided by the initial area% during the preservation test (area%/start area%), indicating the residual ratio from the initial concentration (183.79 nmol/mL) of the long-acting conjugate of the trigonal agonist.

**[Table 11]**

| | Buffering agent | pH | Saccharide | Isotonic agent | Surfactant | Other |
|---|---|---|---|---|---|---|
| #1 | 20 mM sodium acetate | 5.1 | 8% sucrose | - | 0.02% polysorbate 20 | 0.1 mg/mL |
| #2 | 20 mM sodium acetate | 5.1 | 8% sucrose | - | 0.02% polysorbate 80 | 0.1 mg/mL methionin e |
| #3 | 20 mM sodium acetate | 5.1 | 8% sucrose | - | 0.2% poloxamer 188 | 0.1 mg/mL methionin e |

**[Table 12]**

| | IE-HPLC (%) | | | | RP-HPLC (%) | | | |
|---|---|---|---|---|---|---|---|---|
| | Initial | 2 weeks | 4 weeks | 6 weeks | Initial | 2 weeks | 4 weeks | 6 weeks |
| #1 | 100 | 97.3 | 90.8 | 86.0 | 100 | 98.8 | 97.7 | 96.3 |
| #2 | 100 | 97.5 | 91.6 | 87.9 | 100 | 99.2 | 98.5 | 96.8 |
| #3 | 100 | 97.3 | 91.4 | 87.8 | 100 | 99.1 | 98.3 | 96.8 |

The formulations containing each of polysorbate 20 (#1), polysorbate 80 (#2) and poloxamer 188 (#3) as nonionic surfactants showed similar stability.

### Example 6: Evaluation of Stability of Long-Acting Conjugate of Glucagon, GLP-1, and GIP Trigonal Agonist According to Presence or Absence of Nonionic Surfactants and Amino Acids

The stability of long-acting conjugate of glucagon, GLP-1, and GIP trigonal agonist was compared when the liquid formulation contained or did not contain a nonionic surfactant or an amino acid stabilizer.

The long-acting conjugate of the trigonal agonist obtained above was prepared as a liquid formulation using the composition shown in Table 13 (the concentration of the long-acting conjugate is 183.79 nmol/mL) and stored at 25°C for 6 weeks, and then the stability was analyzed by ion-exchange high-performance liquid chromatography (IE-HPLC) and reverse-phase-high-performance liquid chromatography (RP-HPLC).

In Table 14, IE-HPLC (%) and RP-HPLC (%) indicate the percentage of the ratio of the area% at the time of measurement divided by the initial area% during the preservation test (area%/start area%), indicating the residual ratio from the initial concentration (183.79 nmol/mL) of the long-acting conjugate of the trigonal agonist.

**[Table 13]**

| | Buffering agent | pH | Saccharide | Isotonic agent | Surfactant | Other |
|---|---|---|---|---|---|---|
| #1 | 20 mM sodium acetate | 5.1 | 8% sucrose | - | 0.02% polysorbate 20 | 0.1 mg/mL methionin e |
| #2 | 20 mM sodium acetate | 5.1 | 8% sucrose | - | 0.02% polysorbate 20 | - |
| #3 | 20 mM sodium acetate | 5.1 | 8% sucrose | - | - | 0.1 mg/mL methionin e |
| #4 | 20 mM sodium acetate | 5.1 | 8% sucrose | - | - | - |

**[Table 14]**

| | IE-HPLC (%) | | | | RP-HPLC (%) | | | |
|---|---|---|---|---|---|---|---|---|
| | Initial | 2 weeks | 4 weeks | 6 weeks | Initial | 2 weeks | 4 weeks | 6 weeks |
| #1 | 100 | 97.3 | 90.8 | 86.0 | 100 | 98.8 | 97.7 | 96.3 |
| #2 | 100 | 97.0 | 91.0 | 85.8 | 100 | 98.6 | 97.4 | 96.0 |
| #3 | 100 | 97.2 | 91.0 | 85.7 | 100 | 98.6 | 97.5 | 95.7 |
| #4 | 100 | 97.5 | 90.9 | 84.9 | 100 | 99.5 | 97.5 | 95.6 |

As can be seen from the above results, the formulations containing the nonionic surfactant and the amino acid and the formulations without the same showed similar stability.

### Example 7: Evaluation of Stability of Long-Acting Conjugate of Glucagon, GLP-1, and GIP Trigonal Agonist According to Concentrations

Long-term storage stability and accelerated stability were confirmed at the concentrations shown in Table 15 for high-concentration application of the liquid formulation consisting of sodium acetate, sucrose, polysorbate 20, and methionine at pH 5.1 of Example 6.

To this end, after storage at 5°C ± 3°C and 25°C ± 2°C, analysis was performed using ion-exchange high-performance liquid chromatography, reverse-phase-high-performance liquid chromatography, and size-exclusion chromatography. In Tables 16 and 17, IE-HPLC (%), RP-HPLC (%) and SE-HPLC (%) indicate the percentage of the ratio of the area% at the time of measurement divided by the initial area% during the preservation test (area%/start area%), indicating the residual ratio of the long-acting conjugate of the trigonal agonist.

**[Table 15]**

| | Concentratio n of long-acting conjugate | Buffering agent | pH | Saccharide | Isotonic agent | Surfactant | Other |
|---|---|---|---|---|---|---|---|
| #1 | 183.79 nmol/ mL | 20 mM sodium acetate | 5.1 | 8% sucrose | - | 0.02% polysorbate 20 | 0.1 mg/m L methionin e |
| #2 | 551.37 nmol/ mL | | | | | | |

**[Table 16]**

| | | Long-term Storage Stability Test (stored at 5°C ± 3°C) | | | |
|---|---|---|---|---|---|
| | | Initial | 1 month | 3 months | 6 months |
| IE-HPLC (%) | #1 | 100 | 98.8 | 97.2 | 95.2 |
| | #2 | 100 | 98.5 | 96.8 | 94.9 |
| RP-HPLC (%) | #1 | 100 | 99.9 | 99.3 | 98.7 |
| | #2 | 100 | 99.7 | 99.0 | 98.6 |
| SE-HPLC (%) | #1 | 100 | 99.9 | 99.7 | 99.5 |
| | #2 | 100 | 99.9 | 99.7 | 99.2 |

**[Table 17]**

| Accelerated Stability Test (stored at 25°C ± 2°C) | | | | | | | | | | | | |
|---|---|---|---|---|---|---|---|---|---|---|---|---|
| | IE-HPLC (%) | | | | RP-HPLC (%) | | | | SE-HPLC (%) | | | |
| | Initial | 1 mont h | 3 mont hs | 6 mont hs | Initial | 1 mont h | 3 mont hs | 6 mont hs | Initial | 1 mont h | 3 mont hs | 6 mont hs |
| #1 | 100 | 90.7 | 79.3 | 65.9 | 100 | 98.4 | 97.0 | 88.7 | 100 | 98.9 | 97.4 | 95.0 |
| #2 | 100 | 90.3 | 78.4 | 65.3 | 100 | 97.7 | 96.2 | 87.3 | 100 | 98.7 | 97.1 | 93.1 |

As shown in the above results, as a result of the long-term storage stability test, it was confirmed that the long-acting conjugate of glucagon, GLP-1, and GIP trigonal agonist was similarly stable in the liquid formulation of the present invention at protein concentrations of 183.79 nmol/mL to 551.37 nmol/mL. That is, these results suggest that various concentrations of long-acting conjugate of glucagon, GLP-1, and GIP trigonal agonist have stability in the compositions of the liquid formulation of the present invention.

From the foregoing, a skilled person in the art to which the present invention pertains will be able to understand that the present invention may be embodied in other specific forms without modifying the technical concepts or essential characteristics of the present invention. In this regard, the exemplary embodiments disclosed herein are only for illustrative purposes and should not be construed as limiting the scope of the present invention. On the contrary, the present invention is intended to cover not only the exemplary embodiments but also various alternatives, modifications, equivalents, and other embodiments that may be included within the spirit and scope of the present invention as defined by the appended claims.

## Claims

1. A liquid formulation of a long-acting conjugate, wherein the liquid formulation comprises:
18 nmol/mL to 920 nmol/mL of a long-acting conjugate of Chemical Formula 1 below;
a buffering agent in an amount for maintaining the pH of the liquid formulation in the range of 5.0 to 7.0; and
0.5% (w/v) to 10% (w/v) of a sugar alcohol, saccharide, or a combination thereof:
[Chemical Formula 1] Q - Lₐ - Z
In Chemical Formula 1 above,
Q is a peptide of General Formula 1 below;
L is a linker containing an ethylene glycol repeating unit;
a is 0 or a natural number, with the proviso that when a is 2 or more, each L is independent of each other;
Z is an immunoglobulin Fc fragment; and
- represents a covalent bond: 
[General Formula 1]
Xaa1–Aib–Xaa3–Gly–Thr–Phe–Thr–Ser–Asp–Xaa10–Ser–Xaa12–Xaa13–
Xaa14–Xaa15–Glu–Xaa17–Xaa18–Xaa19–Lys–Xaa21–Phe–Val–Xaa24–Trp–Leu–
Leu–Xaa28–Xaa29–Xaa30–R1 (General Formula 1, SEQ ID NO: 47)
in General Formula 1 above,
a lactam ring is formed between the underlined glutamic acid (Glu) at position 16 and the underlined lysine (Lys) residue at position 20 from the N-terminus, wherein
Xaa1 is histidine, 4-imidazoacetyl (CA), or tyrosine;
Xaa3 is glutamic acid or glutamine;
Xaa10 is tyrosine or cysteine;
Xaa12 is lysine or isoleucine;
Xaa13 is tyrosine, alanine, or cysteine;
Xaa14 is leucine or methionine;
Xaa15 is cysteine or aspartic acid;
Xaa17 is arginine, isoleucine, cysteine, or lysine;
Xaa18 is alanine, arginine, or histidine;
Xaa19 is alanine, glutamine, or cysteine;
Xaa21 is glutamic acid, or aspartic acid;
Xaa24 is glutamine, asparagine, or aspartic acid;
Xaa28 is alanine, asparagine, or aspartic acid;
Xaa29 is cysteine, glycine, glutamine, threonine, glutamic acid, or histidine;
Xaa30 is cysteine, glycine, lysine, or histidine, or is absent; and
R1 is cysteine, m-Ser-Ser-Gly-Ala-Pro-Pro-Pro-Ser-n (SEQ ID NO: 48), or m-Ser-Ser-Gly-Gln-Pro-Pro-Pro-Ser-n (SEQ ID NO: 49), or is absent,
wherein:
m is Cys, or Pro; and
n is Cys, or Gly, or is absent.

2. The liquid formulation of claim 1, wherein the peptide is any one amino acid sequence selected from SEQ ID NOS: 1 to 46.

3. The liquid formulation of claim 1, wherein the peptide is any one amino acid sequence selected from SEQ ID NOS: 1, 2, 9, 19, 21 to 27, 30 to 32, or 40 to 46.

4. The liquid formulation of claim 1, wherein the peptide is any one amino acid sequence selected from SEQ ID NO: 9, 30 to 32, or 42 to 46.

5. The liquid formulation of claim 1, wherein the peptide is SEQ ID NO: 9.

6. The liquid formulation of claim 1, wherein R1 is cysteine, Cys-Ser-Ser-Gly-Gln-Pro-Pro-Pro-Ser (SEQ ID NO: 50), Pro-Ser-Ser-Gly-Ala-Pro-Pro-Pro-Ser (SEQ ID NO: 51), Pro-Ser-Ser-Gly-Ala-Pro-Pro-Pro-Ser-Gly (SEQ ID NO: 52), Pro-Ser-Ser-Gly-Gln-Pro-Pro-Pro-Ser (SEQ ID NO: 53), or Pro-Ser-Ser-Gly-Gln-Pro-Pro-Pro-Ser-Cys (SEQ ID NO: 54), or is absent.

7. The liquid formulation of claim 1, wherein Q is amidated at the C-terminus thereof.

8. The liquid formulation of claim 1, wherein Q is linked via a sulfur atom of cysteine.

9. The liquid formulation of claim 1, wherein the immunoglobulin Fc fragment is derived from IgG4.

10. The liquid formulation of claim 1, wherein Z is a structure in which two polypeptide chains are linked by a disulfide bond, and are linked only through a nitrogen atom in one of the two chains.

11. The liquid formulation of any one of claims 1 to 10, wherein Z is a homodimer of the amino acid sequence of SEQ ID NO: 76.

12. The liquid formulation of claim 11, wherein Z is linked through a nitrogen atom of proline at the N-terminus thereof.

13. The liquid formulation of claim 1, wherein the immunoglobulin Fc fragment and Q are non-glycosylated.

14. The liquid formulation of claim 1, wherein L is polyethylene glycol.

15. The liquid formulation of claim 1, wherein the formula weight of the ethylene glycol repeating unit moiety in L is in the range of 1 kDa to 100 kDa.

16. The liquid formulation of claim 1, wherein the buffering agent is selected from the group consisting of citric acid and a salt thereof, acetic acid and a salt thereof, histidine and a salt thereof, phosphoric acid and a salt thereof, and a combination thereof.

17. The liquid formulation of claim 14, wherein the buffering agent is acetic acid and a salt thereof.

18. The liquid formulation of claim 1, wherein the pH of the liquid formulation is 5.0 to 5.5.

19. The liquid formulation of claim 1, wherein the pH of the liquid formulation is 5.0 to 6.5.

20. The liquid formulation of claim 1, wherein the pH of the liquid formulation is 5.1 to 6.0.

21. The liquid formulation of claim 20, wherein the pH of the liquid formulation is 5.1 to 5.5.

22. The liquid formulation of claim 1, wherein the concentration of the buffering agent is 5 mM to 100 mM for maintaining the pH of the liquid formulation in the range of 5.0 to 7.0.

23. The liquid formulation of claim 1, wherein the saccharide is glucose, fructose, galactose, lactose, maltose, sucrose, or a combination thereof.

24. The liquid formulation of claim 23, wherein the saccharide is sucrose.

25. The liquid formulation of claim 1, wherein the sugar alcohol is one or more selected from the group consisting of mannitol and sorbitol.

26. The liquid formulation of claim 1, wherein the liquid formulation further comprises one or more components selected from the group consisting of a nonionic surfactant and an amino acid.

27. The liquid formulation of claim 26, further comprising an isotonic agent.

28. The liquid formulation of claim 26, wherein the nonionic surfactant is contained at a concentration of 0.01% (w/v) to 0.1% (w/v) in the liquid formulation.

29. The liquid formulation of claim 26, wherein the nonionic surfactant is poloxamer, polysorbate, or a combination thereof.

30. The liquid formulation of claim 29, wherein the nonionic surfactant is selected from the group consisting of poloxamer 188, polysorbate 20, polysorbate 40, polysorbate 60, polysorbate 80, and a combination thereof.

31. The liquid formulation of claim 26, wherein the amino acid further comprises a stabilizer selected from the group consisting of arginine, glycine, methionine, and a combination thereof.

32. The liquid formulation of any one of claims 1 to 31, wherein the liquid formulation comprises:
90 nmol/mL to 552 nmol/mL of a peptide conjugate of Chemical Formula 1;
5 mM to 25 mM of a buffering agent selected from citric acid and a salt thereof, acetic acid and a salt thereof, histidine and a salt thereof, phosphoric acid and a salt thereof, and a combination thereof so that the pH of the liquid formulation is 5.0 to 6.5;
1% (w/v) to 10% (w/v) of a sugar alcohol, saccharide, or a combination thereof;
0.01% (w/v) to 0.1% (w/v) of a nonionic surfactant selected from poloxamer, polysorbate, or a combination thereof; and
0.01 mg/mL to 1 mg/mL of a stabilizer selected from the group consisting of arginine, glycine, methionine, and combinations thereof.

33. The liquid formulation of any one of claims 1 to 31, wherein the liquid formulation comprises:
90 nmol/mL to 552 nmol/mL of a peptide conjugate of Chemical Formula 1;
5 mM to 25 mM of a buffering agent selected from citric acid and a salt thereof, acetic acid and a salt thereof, histidine and a salt thereof, phosphoric acid and a salt thereof, and a combination thereof so that the pH of the liquid formulation is 5.0 to 5.5;
4% (w/v) to 10% (w/v) of a saccharide;
0.01% (w/v) to 0.1% (w/v) of a nonionic surfactant selected from poloxamer, polysorbate, or a combination thereof; and
0.01 mg/mL to 1 mg/mL of a stabilizer selected from the group consisting of arginine, glycine, methionine, and combinations thereof.

34. The liquid formulation of any one of claims 1 to 25, wherein the liquid formulation comprises:
90 nmol/mL to 552 nmol/mL of a peptide conjugate of Chemical Formula 1;
5 mM to 25 mM of a buffering agent selected from citric acid and a salt thereof, acetic acid and a salt thereof, histidine and a salt thereof, phosphoric acid and a salt thereof, and a combination thereof so that the pH of the liquid formulation is 5.0 to 5.5; and
4% (w/v) to 10% (w/v) of a saccharide.

35. The liquid formulation of claim 34, further comprising 0.01 mg/mL to 1 mg/mL of a stabilizer selected from the group consisting of arginine, glycine, methionine, and a combination thereof.

36. The liquid formulation of claim 34, further comprising 0.01% (w/v) to 0.1% (w/v) of a nonionic surfactant selected from poloxamer, polysorbate, or a combination thereof.

37. The liquid formulation of claim 35, further comprising 0.01% (w/v) to 0.1% (w/v) of a nonionic surfactant selected from poloxamer, polysorbate, or a combination thereof.
